# EUROPÄISCHE PATENTSCHRIFT

(11) **EP 4 329 835 B1**
(45) Veröffentlichungstag und Bekanntmachung des Hinweises auf die Patenterteilung: **09.07.2025**
(21) Anmeldenummer: 22726172.4
(22) Anmeldetag: 28.04.2022
(51) Int. Cl.: A61L 29/12, A61F 5/445

(54) **KATHETERARTIGE VORRICHTUNG FÜR DIE ZU- UND/ODER ABLEITUNG VON SUBSTANZEN ZU BZW. AUS DEM KÖRPER EINES PATIENTEN UND VERFAHREN ZUR HERSTELLUNG EINER DERARTIGEN VORRICHTUNG**
CATHETER-LIKE DEVICE FOR THE SUPPLY AND/OR REMOVAL OF SUBSTANCES TO OR FROM THE BODY OF A PATIENT AND METHOD FOR THE MANUFACTURE OF SUCH A DEVICE
DISPOSITIF DE TYPE CATHÉTER POUR L'APPORT ET/OU L'ÉVACUATION DE SUBSTANCES VERS OU DEPUIS LE CORPS D'UN PATIENT ET PROCÉDÉ DE FABRICATION D'UN TEL DISPOSITIF

(30) Priorität: 28.04.2021 DE 102021002240
(43) Veröffentlichungstag der Anmeldung: 06.03.2024
(73) Patentinhaber: Advanced Medical Balloons GmbH, 68753 Waghäusel (DE)
(72) Erfinder: GÖBEL, Fred, 67346 Speyer (DE)
(74) Vertreter: Küchler, Stefan
(86) Internationale Anmeldenummer: PCT/IB2022/053956
(87) Internationale Veröffentlichungsnummer: WO 2022/229899

(56) Entgegenhaltungen:
- WO-A1-2011/142928
- WO-A1-2013/106361

## Beschreibung

Die Erfindung richtet sich auf besonders einfache Bauweisen zu- und/oder ableitender, im Körper verweilender Kathetersysteme, sowie auf ein Verfahren zu deren Herstellung.

Insbesondere richtet sich die Erfindung auf eine Vorrichtung zur minimal irritierenden, gewebeverträglichen, sich bevorzugt an die Bewegungen des Körpers und seiner Organe anpassenden Zu- und/oder Ableitung von Substanzen, umfassend einen in einem Binnenraum des Körpers platzierbaren, von außerhalb des Körpers befüllbaren Ballon, der - ggf. zusammen mit einem zu- und/oder ableitenden, den Ballon tragenden Schlauch- oder Schaftsegment - ein befüllbares Kompartiment umschließt, an welches sich ein schlauch- oder schaftförmiges Segment anschließt, das den Binnenraum mit der Körperoberfläche verbindet, wobei der Ballon durch Blasformen aus einem Schlauchrohling aus einem mehrlagigen, folienartigen Material hergestellt ist, sowie auf ein Verfahren zur Herstellung einer derartigen Vorrichtung.

Bei Ballonkathetern, die für das längerfristige Verbleiben im Körper eines Patienten konzipiert sind, ist es erforderlich, dass das jeweilige, vom Ballon aufgenommene Füllmedium über den gesamten Verlauf der Anwendung hinweg möglichst volumenkonstant im Ballon gehalten werden kann. Abhängig von den physikalischen und chemischen Eigenschaften des Ballonmaterials, können flüssige oder gasförmige Medien im Anwendungsverlauf aus der Ballonkomponente in den Körper bzw. dessen jeweiligen Milieus austreten. Eine durch einen derartigen, spontanen Volumenverlust verursachte Entleerung des Ballons geht in der Regel mit einem partiellen oder vollständigen Verlust der Funktion des Katheters oder der Vorrichtung einher.

Insbesondere aus Silikon bestehende Ballonkomponenten sind häufig auch bei höheren Wandungsstärken nur in sehr eingeschränktem Umfang fähig, eine flüssige oder auch gasförmige Füllung, beispielsweise mit Wasser oder Luft, zuverlässig volumenkonstant im Ballon zu halten. Auch bei latexbasierten Ballons sind spontane Volumenverluste im Anwendungsverlauf nicht ungewöhnlich.

Sowohl bei Silikonen, als auch bei latexartigem Naturkautschuk, erklären sich die Volumenverluste in der Regel durch eine dem Material immanente, typische Porosität, wobei die Durchlässigkeit der porösen Wandung mit wachsendem Dehnungszustand der Ballonhülle zunimmt.

WO2011/142928 A offenbart ein geschlossenes System für die möglichst geruchsneutrale Zu- und/oder Ableitung von geruchsintensiven Substanzen zu/ von dem Körper eines Patienten, umfassend eine in einem Binnenraum des Körpers des Patienten positionierbare, ein ballonartiges Element aufweisende, das System im Körper des Patienten retinierende und/oder dichtende Kopfeinheit, einen extrakorporal angeordneten Behälter mit einem bevorzugt folienbasierten, beutelartigen Kompartiment für die Aufnahme der zu- und/oder abzuleitenden Substanzen, sowie eine schlauchfolienartige Struktur, die den zu erreichenden Körperinnenraum mit dem extrakorporal angeordneten Behälter verbindet, wobei die folienbasierten Bestandteile des Behälters aus mehrlagigem Folienmaterial bestehen, das jeweils wenigstens eine als Geruchsbarriere wirkende Barrierelage aus Ethylen-Vinylalkohol-Copolymer (EVOH) und/oder aus Polyvinylidenchlorid (PVDC) aufweist, sowie jeweils wenigstens eine Trägerlage aus einem robusten, mechanisch belastbaren Material. Das ballonartige Element kann z.B. aus Polyurethan bestehen.

Ballonmaterialien mit einer ausgeprägten molekularen . Polarität, wie beispielsweise Polyurethan, weisen zwar keine poröse Wandstruktur auf, zeigen aber bei der Befüllung mit polaren Medien kleiner Molekülgröße, wie insbesondere Wasser, dennoch eine hohe Durchlässigkeit. Das polar geladene Wasser tritt in das in das ebenfalls polar geladene PUR-Material der Ballonhülle ein und tritt bei entsprechend wirksamen Konzentrationsgradienten auf der anderen Seite der Ballonwandung wieder aus. Die "Lösung" bzw. Migration von Wasser durch eine PUR-basierte Ballonhülle, kann bei bestimmten Bedingungen im Inneren des Ballons zur Akkumulation von Wasser führen, was beispielsweise bei trachealen Beatmungskathetern deren Funktion und Sicherheit risikorelevant beeinträchtigen kann. Es ist bekannt, dass Wasser-Tröpfchen, die in die kleinlumige, zum Ballon (Cuff) eines Trachealtubus führende Zuleitung gelangen, eine passagere Unterbrechung der freien Verbindung zwischen dem tracheal dichtenden Ballon und einer druckmessenden oder druckkontrollierenden Vorrichtung außerhalb des Körpers verursachen können.

Die Migration von Wasser durch PUR basierte Ballonhüllen kann ferner bei der Einhausung wassersensibler, elektronischer Mess- oder Sensorkomponenten problematisch sein.

Wird ein PUR-basierter Ballon in einer wässrigen Lösung bzw. in wässrigen Körperflüssigkeiten platziert, die einen osmotisch wirksamen Gradienten zum wässrigen Füllmedium des Ballons aufweisen, können sich über den Ballonmantel hinweg konzentrationsabhängige Verschiebungen von Wasser aus dem Ballon heraus, in das den Ballon umgebende Milieu einstellen. Beispielsweise kann es bei der Platzierung eines mit Wasser befüllten PUR-Ballons in der Harnblase zu einer solchen, osmotisch angetriebenen Migration von Wasser aus dem Katheterballon, in den mit Salzen und organischen Molekülen befrachteten Urin kommen.

Die spontane Entleerung eines mit Gas, beispielsweise mit Luft befüllten Katheterballons in ein den Ballon umgebendes Körpermedium stellt bei besonders dünnwandig hergestellten Ballonmaterialien eine besondere Herausforderung dar. Bei der Platzierung eines aus PUR bestehenden, mikrodünnwandigen Ballons in der Harnblase eines Patienten stellen sich in der Regel innerhalb weniger Tage Reduktionen des Füllvolumens ein, die zum weitgehenden Verlust der retinierenden Funktion des Katheterballons führen können.

Ein gleich gelagerter Gesichtspunkt tritt bei Schläuchen für die Zu- und/oder Ableitung von Medien in den bzw. aus dem menschlichen Körper auf, wenn diese Medien den betreffenden Schlauch nicht durch dessen Wandung verlassen sollen, bspw. um eine Geruchsbelästigung zu vermeiden.

Die Durchlässigkeit der jeweiligen Ballonhülle für wässrige und gasförmige Medien korreliert neben deren molekularen Polarität vor allem mit deren Wandstärke. Je dünnwandiger die Ballonhülle ist, desto leichter treten die jeweiligen Medien über. Bei vielen Anwendungen von Katheterballons hängt die spezifische Katheterfunktion jedoch in besonderem Maße von maximal niedrigen Wandungsstärken der Ballonhülle ab. Die langfristige Platzierung im Körper erfordert bei derartigen, membranartig dünnwandigen Ballons daher in der Regel eine mehr oder weniger engmaschig intermittierende Kontrolle und Korrektur des für die Funktion erforderlichen Füllvolumens. Vom Anwender werden daher Ballonfolien erwartet, deren besonderer Folienaufbau bzw. deren Kombination von Einzellagen mit jeweils spezifischen, physikalischchemischen Eigenschaften, die spontane Entleerung des Ballons durch Migrations- und Osmose-Effekte verhindert.

Daraus folgt das die Erfindung initiierende Problem, eine katheterartige, zu- oder ableitende Vorrichtung, die im Körper prolongiert verweilt, bevorzugt für die kombinierte Retention und Dichtung der Vorrichtung ausgestattet ist und/oder über einen besonderen trans-luminal dichtenden Aufrichtungsmechanismus verfügt, derart auszubilden, dass er auf wirtschaftlich vorteilhafte Weise hergestellt werden kann und über einen längeren Zeitraum hinweg verwendbar ist und dabei ggf. auch Veränderungen des Körperlumens in einer sich optimal adaptierenden Weise folgen kann.

Die Lösung des gestellten Problems gelingt bei einer gattungsgemäßen, katheterartigen, zu- oder ableitenden Vorrichtung dadurch, dass ein in einem Binnenraum des Körpers platzierbarer, von außerhalb des Körpers befüllbarer Ballon, der - ggf. zusammen mit einem zu- und/oder ableitenden, den Ballon tragenden Schlauch- oder Schaftsegment - ein befüllbares Kompartiment umschließt, an welches sich ein schlauch- oder schaftförmiges Segment anschließt, das den Binnenraum mit der Körperoberfläche verbindet, wobei der Ballon durch Blasformen aus einem Schlauchrohling aus einem mehrlagigen, folienartigen Material hergestellt ist, wobei der Schlauchrohling mehrlagig koexdrudiert ist, und in dem mehrlagigen Material zusätzlich zu wenigstens einer elastisch verformbaren Lage aus Polyurethan (PUR) wenigstens eine nicht elastisch verformbare, geruchs- und/oder mediendichte Barriere-Lage aus Ethylen-Vinylalkohol-Copolymer (EVOH) oder aus Polyvinylidenchlorid (PVCD), oder aus Polyamid (PA) oder aus einem thermoplastischen Polyamidelastomer (TPE-A) vorgesehen ist, wobei die verschiedenen Lagen durch Koextrusion hergestellt sind, und wobei die Gesamtstärke aller elastisch verformbaren Lagen wenigstens dem 1,5-fachen der Gesamtstärke aller nicht elastisch verformbaren, geruchs- und/oder mediendichte Barriere-Lagen entspricht.

Damit beschreibt die vorliegende Erfindung einfache technische Lösungswege zur herstellungsoptimierten, kostengünstigen Erzeugung von katheterartigen, zu- oder ableitenden Vorrichtungen, die im Körper prolongiert verweilen, und insbesondere für die kombinierte Retention und Dichtung der Vorrichtung ausgestattet sind.

Zur Vermeidung spontaner Entleerungen besonders dünnwandiger Ballonwandungen schlägt die Erfindung einen mehrlagigen Aufbau der Folienwandung vor, wobei eine spezifische Materiallage mit besonderen gas- und/oder flüssigkeitsdichten Trenneigenschaften in die Ballonhülle integriert wird. Insbesondere kommen EVOH-basierte Materialien für die Herstellung solcher Barriere-Eigenschaften in Frage. EVOH oder auch als EVAL (Ethylen-Vinylalkohol Copolymer) bezeichnet weist bereits in sehr niedrigen Schichtdicken von beispielsweise 4 bis 10 Mikrometer eine sehr hohe, gasdichtende Effizienz auf, und bietet zudem einen effizient dichtenden Effekt auf die Permeation bzw. Migration von Wasser bzw. Wasserdampf. Neben der effizienten Barrierewirkung ist EVOH als wesentliche weitere Voraussetzung für die Anwendung im Rahmen der vorliegenden Erfindung ausreichend gut plastisch verformbar, um im Verbund mit beispielsweise aus PUR bestehenden Schichtlagen in eine definierte, ballonartige Form aus- bzw. umgeformt werden zu können.

Für die Formung besonders dünnwandiger Folienkörper aus PUR, ist extrudiertes Schlauchmaterial erforderlich, dass in der Regel in einem separaten, der Blasformung vorausgehenden Fertigungsschritt hergestellt wird. Das im Rahmen der Erfindung eingesetzte Folienmaterial ist im bevorzugten Falle 3-lagig, wobei die äußere und die innere Schlauchlage aus PUR bestehen und die EVOH basierte Trennlage in mittiger Position zwischen den beiden Lagen PUR-Lagen aufgenommen wird. Im bevorzugten Falle wird bei dieser Materialkombination auf haftvermittelnde Zwischenlagen, die das PUR und dem EVOH mechanisch beständig und ohne Delamination der Einzellagen verbinden, verzichtet.

Ein weiteres barriere-wirksames Material ist PVDC (Polyvinyldienchlorid). Es weist gleich effiziente Sauerstoff- und Wasserdampf dichtende Eigenschaften auf, und ist somit im Rahmen der Erfindung eine bevorzugte, mehrfachfunktionelle Trennschicht. Es kann ferner mit beispielsweise PUR mehrlagig als Rohschlauch extrudiert und durch Blasformung des Schlauchrohlings plastisch umgeformt bzw. "ausgezogen" werden. Die für die Barriere erforderlichen Wandstärken liegen wie bei EVOH im einstelligen Mikrometerbereich.

Zur Erreichung mikro-dünnwandiger, durch Blasformung hergestellter Ballonhüllen aus PUR oder PUR-enthaltenden Materialien, wird der in einem separaten Prozess extrudierte Rohschlauch einer gewissen axialen Streckung unterzogen, anschließend in einer formenden Kavität erhitzt und durch Beaufschlagung mit Blasdruck zu einem ballonartigen Körper verformt bzw. in diese Kavität hinein expandiert. Die axiale und radiale Streckung des Schlauchmaterials verleiht dem ausgeformten Ballon eine gewisse polymere Orientierung, die ihm hohe mechanische Festigkeit und Formstabilität verleiht. Im Moment der kombinierten axialen und radialen Expansion des Rohschlauches zum Ballon stellt sich ein, mit der jeweiligen Streckung korreliertes, parallelisiertes Ausrichtungsmuster der amorphen Anteile des Polymers ein, wobei sich diese anteilig linear ausrichten. Die Ausrichtung der polymeren Ketten wird durch die anschließende Kühlung im umgeformten Zustand fixiert, wobei bis auf etwa 5 bis 10 % Retraktion die Formmasse des Ballons im erhitzten Zustand erhalten bleibt.

Die erfindungsgemäße Technologie beschreibt in einer einfachsten Ausführungsform die durchgängige Ausformung aller die Kopfeinheit der Vorrichtung umfassenden Anteile aus einem einzigen Schlauchrohling. In Erweiterung der Ausformung der Kopfeinheit aus einem Rohling, kann auch die sich der Kopfeinheit nach extrakorporal hin anschließende ableitende Schlaucheinheit im gleichen Arbeitsschritt mit der Kopfeinheit ausgeformt werden. Die erfindungsgemäße Besonderheit bei der Herstellung des Kopfteils besteht in der spezifischen Ausführung und Anordnung, mit einem welligen Profil versehener (korrugierter) Segmente im Bereich des intra-korporalen, trans-luminalen Segmentes der Vorrichtung. Die spezifische Profilgebung des intrakorporal retinierenden und/oder trans-luminal zu- oder ableitenden Schlauches gewährleistet, dass sowohl innerhalb des retinierenden Segmentes, z.B. im Rektum, als auch innerhalb des trans-luminalen Segmentes, z.B. im Analkanal, eine ausreichend starke elastische Selbstaufrichtung des zu- und/oder ableitenden Lumens generiert wird, so dass z.B. Darminhalt möglichst unbehindert nach außen abfließen kann.

Indem die zu- und oder ableitende Schlauchstruktur, welche den im jeweiligen Binnenraum oder Organ retinierenden Ballon oder Ballonanteil trägt, und/oder das den Zugangsweg zum Binnenraum bildende, trans-luminale Segment der Vorrichtung eine wellig ausgeformte, ring- oder wendelartige Profilgebung aufweist, verfügt die Vorrichtung über einen besonderen trans-luminal dichtenden Aufrichtungsmechanismus, der Veränderungen des Analkanals in einer sich optimal adaptierenden Weise folgt und anpasst. Die Erfindung geht insbesondere auf den Aspekt der wirtschaftlich vorteilhaften Herstellbarkeit ein, wobei sämtliche, für die Herstellung der trans-anal positionierten Kopfeinheit der Vorrichtung erforderlichen Komponenten vorzugsweise aus einem einzigen, in die Herstellung eingesetzten Schlauchrohling in einem einzigen Arbeitsgang erzeugt werden.

Die jeweilige Einstellung der elastischen Verformungs- und Selbstaufrichtungseigenschaften der intrakorporalen, insbesondere rektalen, und der transluminalen, insbesondere trans-analen, Schlauchanteile erfolgt zum einen durch die elastischen Eigenschaften des verwendeten Materials und dessen Wandungsstärke, zum anderen durch die jeweilige geometrische Ausführung des Wellinngsprofils.

Die durch die Profilgebung unterstützten Aufrichtungseigenschaften ermöglichen es in einer für die atraumatische Anwendung besonders vorteilhaften Weise die Schlauchwandstärken in diesem Segment in den folienartig dünnen Bereich zu reduzieren. Die Wahrscheinlichkeit struktureller und funktioneller Schädigungen z.B. des analen Schließmuskels kann so reduziert werden. Die erfindungsgemäße Kombination von Material und Formgebung ermöglicht es ferner die mechanischen Verformungs- und Aufrichtungseigenschaften innerhalb enger Grenzen einstellbar bzw. reproduzierbar herstellbar zu machen.

Beschrieben werden zum einen Wandungsprofile, wie sie typischerweise durch Blasformungsprozesse hergestellt werden, wobei ein zuvor extrudierter, relativ dickwandiger Rohschlauch, in einen relativ dünnwandigen Folienschlauch umgeformt wird bzw. ein relativ kleinlumiger Rohschlauch durch Beaufschlagung mit Blasdruck auf einen relativ größeren Arbeitsdurchmesser expandiert, und im erhitzten Zustand in eine entsprechend profilierte Formwandung hinein geschmiegt wird. Die Erfindung schließt vergleichbare Profilstrukturen ein, wie sie beispielsweise auch durch einen ein- oder mehrschichtigen Tauchprozess oder durch einen Spritzgussprozess hergestellt werden können.

Die im Rahmen der Erfindung eingesetzten Schlauchrohlinge bestehen vorzugsweise aus mehrlagigem Material, wobei Materiallagen mit elastischen Verformungscharakteristika, wie sie bevorzugt thermoplastischen Polyurethane (TPU) bieten, anteilig überwiegen.

In den mehrschichtigen Lagenaufbau können, für die jeweilige Funktion des Katheters vorteilhaft, besonders geruchsdichte bzw. mediendichte Barrierelagen integriert sein. Der Schlauchfolienrohling kann beispielsweise einen sandwichartigen Aufbau aus PUR-Lagen auf den innen und außen liegenden Seiten sowie einer mittigen Lage aus EVOH oder PVCD aufweisen. In Ergänzung zum elastisch wirkenden PUR können auch weitere nichtelastische Materialien ergänzt werden, sofern diese beispielsweise für die Erreichung bestimmter Oberflächeneigenschaften im Produkt erforderlich sind. Optional zu elastischen Materialien, kommen, weniger bevorzugt, optional ebenfalls PVC- und PE-basierte Materialtypen zum Einsatz.

Im Falle einer integrierten Ausformung des sich an die Kopfeinheit anschließenden, ab- und/oder zuleitenden, extrakorporalen Schlauchelementes, wird das entsprechende Schlauchelement bevorzugt derart dünnwandig ausgeführt, so dass es sich bei Krafteinwirkung von außen radial nach innen gerichtet einstülpt oder auch zu einer flachen, bandartigen Struktur kollabiert, und so der Entstehung von druckbedingten Läsionen in der vorbeugt, sollte beispielsweise der Körper des Patienten dem Schlauch zeitweise aufliegen. Das flach kollabierte Schlauchsegment richtet sich in der bevorzugten Ausführungsform der Vorrichtung bei nachlassender Krafteinwirkung von außen, spontan-elastisch auf und erreicht einen zumindest teilweise offenen, partiell gerundeten Querschnitt. Ein entsprechendes Aufrichtungsverhalten kann in idealer Weise wiederum durch eine ein-lagige oder mehrlagige Lagenkomposition aus Polyurethanen hergestellt werden.

PUR ermöglicht wegen seiner hohen mechanischen Festigkeit und relativ geringen Material-Compliance eine besondere Formstabilität der jeweils auf das vollständige Arbeitsmaß ausgeformten Strukturen. Dies erschließt insbesondere die Option der Verwendung kompressibler, gasförmiger Füllmedien, z.B. die Verwendung von Luft. Ferner gestatten derartige, PUR-basierte Ballonkomponenten die Option zur spannungslos schlaffen Befüllung des Ballons, da die ankernde bzw. retinierende Stabilität der Ballonform nicht durch eine kontinuierliche, pralle Dehnung des Ballonmantels hergestellt werden muss, wie dies beispielsweise bei silikonbasierten Ballonkomponenten erforderlich ist. Der PUR-basierte, spannungslos schlaff befüllte Ballonkorpus nimmt die jeweilige, auf ihn einwirkende Kraft auf und geht nur bei von außen auf den Ballon einwirkender Belastung in seine, bei der Herstellung vorgegebene Form über. Bedingt durch die bevorzugt geringe Dehnbarkeit der Ballonhülle, bleibt die jeweilige funktionsrelevante, z.B. retinierende Form des Ballons, bei einer entsprechenden physiologischen Druckbelastung oder auch vom Patienten weg gerichteten Zugbelastung erhalten.

Bei der Verwendung von PUR-Trägerlagen kommen im Rahmen der Erfindung bevorzugt thermoplastische, ester- und ätherbasierte Polyurethane (TPU) der zum Einsatz. Bevorzugt werden Shore Härten in den Bereichen 80A bis 95A, sowie der Härtebereich von 55D bis 60D. Verwendet werden Beispielsweise TPU-Typen der Hersteller Lubrizol (Pellethane . 2363-Series) oder BASF (Elastollan 1100-Series).

Werden PVC-basierte Trägerlagen mit einer EVOH oder PVDC basierten Barriere-Schicht kombiniert, sind die zuvor beschriebenen, PUR-typischen Aufrichtungseigenschaften nur grenzwertig suffizient bzw. nicht erreichbar. Bei einer anteiligen Verwendung von PVC, als beispielsweise tragende Innen- oder Außenlage im mehrlagigen Schlauchmantel, kann die Fähigkeit zur Selbstaufrichtung der rektalen und trans-analen Schlauchanteile jedoch durch Integration einer zusätzlichen PUR Lage, in den Folienaufbau aufgenommen werden. Das verwendete PUR weist dann vorzugsweise eine höhere Shore-Härte auf, im Bereich von beispielsweise Shore 95A, oder auch Shore 55D bis 65D, und kann, um eventuell gewünschte Vorteile von PVC zu erhalten, zur PVC-Lage relativ dünnwandig ausgeführt sein.

Eine nach innen, zum Drainagelumen hin gerichtete PVC-Lage kann im Rahmen der Erfindung konzeptionell von Vorteil sein, da der PVC-typische Barriereeffekt gegen Wasser den erreichbaren Barriereeffekt einer PUR-Lage gleicher Wandungsstärke deutlich übersteigt. Insbesondere bei der Verwendung von EVOH als Barrierelage ist ein möglichst effizienter Schutz vor Wassermolekülen von Vorteil, da dessen Barriereeffizienz durch Exposition mit Wasser reduziert wird.

PUR-basierte Materiallagen statten die im Rahmen der Erfindung beschriebenen, mehrlagig aufgebauten Schlauchkomponenten mit besonderer mechanischer Stabilität aus. Bereits dünnwandige, anteilige PUR-Lagen im Bereich von 10 bis 30 µm verleihen den ausgeformten Schlauch- und Ballonsegmenten ausreichende Zug- und Reißfestigkeit sowie Schnitt- und Punktionsresistenz. Ferner stabilisieren entsprechende PUR-Lagen den Rohling bei der Umformung des Rohlings in das formgebende Werkzeug.

Neben EVOH oder PVDC können als wirksame Geruchsbarriere auch Lagen von Polyamid (PA) oder Pebax, einer dem Polyamid verwandten Substanz, in eine mehrlagige Folie verbaut werden. Die Erfindung schlägt in der bevorzugten Ausführung dünnwandige Polyamid- oder TPE-A Lagen (z.B. Pebax^{®}) von ca. 10 bis 20 µm vor, die mit einer PUR-Trägerlage kombiniert werden. Die erreichbare Barriereeffizienz ist der von EVOH oder PVDC unterlegen.

Die im Rahmen der Erfindung beschriebenen Folienkombinationen können beispielsweise durch mehrlagige Folienextrusion hergestellt werden. Dabei kann der in die Formung eingesetzte Schlauchrohling primär extrudiert werden, oder auch aus mehrlagiger Flachfolie zum blasformbaren Schlauchrohling verarbeitet werden.

Weitere Merkmale, Eigenschaften, vorteile und Wirkungen der Erfindung ergeben sich aus der folgenden Beschreibung bevorzugter Ausführungsformen der Erfindung sowie anhand der Zeichnung. Hierbei zeigt:
- Fig. 1a: einen beispielhaft durch Blasformung aus einem Schlauchrohling ausgeformten Ballonfolienschlauch für die Herstellung einer erfindungsgemäßen Katheter-Kopfeinheit;
- Fig. 1b: den in Fig. 1a dargestellten Ballonfolienschlauch im rückgestülpten, zum befüllbaren Kompartiment geschlossenen Zustand;
- Fig. 1c: eine Kopfeinheit, auf der Basis des in Fig.1a gezeigten Rohlings, wobei neben den Segmenten der Kopfeinheit auch das extrakorporal zu- und/oder ableitende Schlauchsegment aus einem durchgängigen Schlauchrohling ausgeformt wird;
- Fig. 2a: eine Ausformung eines Schlauchrohrings, bei dem sowohl der das gesamte zu- und oder ableitende, intrakorporale Segment mit einer welligen oder wendelartigen Profilgebung versehen ist und das ausgeformte, retinierende Ballonsegment optional durch den Zugangsweg zum jeweiligen Binnenraum hindurch oder auch über diesen hinausreicht;
- Fig. 2b: den in Fig. 2a dargestellten Ballonfolienschlauch im rückgestülpten, zum befüllbaren Kompartiment geschlossenen Zustand;
- Fig. 3: einen ausgeformten Folienschlauch, die dem das intra-korporale Schlauchsegment Bereiche verschieden gestalteter Profilgebung umfasst;
- Fig. 4a: einen ausgeformten Folienschlauch, der im ballontragenden Segment, mit einer zusätzlichen, das Lumen der Vorrichtung stabilisierenden, ring- oder schlauchförmigen Komponente ausgestattet ist;
- Fig 4b: den in Fig. 4a dargestellten Ballonfolienschlauch im rückgestülpten, zum befüllbaren Kompartiment geschlossenen Zustand;
- Fig. 5: ein beispielhaftes, erfindungsgemäßes Wellungsprofi auf der Grundlage eines Schaftschlauches mit 12 bis 14 mm Innendurchmesser;
- Fig. 6.: einen beispielhaften, mehrlagigen Aufbau eines Schlauchfolienrohling;
- Fig. 7: einen weiteren beispielhaften, mehrlagigen Aufbau eines Schlauchfolienrohlings;
- Fig. 8: den beispielhaften, schematischen Aufbau der Wandung eines für die Blasformung eingesetzten Rohschlauches, wobei dieser eine besondere Kombination dreier konzentrisch extrudierter Materiallagen mit einer mittig angeordneten Trennlage aufweist;
- Fig. 9: einen entsprechenden, aus dem in Fig. 8 beschriebenen Rohschlauchtypen, durch ein Blasformungsverfahren ausgeformten, dreilagigen Ballonkörper;
- Fig. 10: einen Schnitt durch einen Ballonkörper gemäß einer weiteren Ausführungform der Erfindung, der über eine besondere, zusätzliche Innenlage aus TPE-basiertem Material verfügt, teilweise abgebrochen;
- Fig. 11: einen Ballonkörper mit einem besonderen, vierlagigen Aufbau in einer der Fig. 10 entsprechenden Ansicht;
- Fig. 12: einen Ballonkörper mit einer besonderen Kombination eines thermoplastischen Polyurethans und zwei zusätzlichen Barriere-Lagen in einer der Fig. 10 entsprechenden Ansicht;
- Fig. 13: einen erfindungsgemäßen Aufbau einer Ballonhülle aus zwei kombinierten Materiallagen in einer schematischen, teilweise abgebrochenen Schnittdarstellung;
- Fig. 14: eine sekretleitende, kanalartige Formation, wie sie sich bei im Umfang residual bemessenen, dichtenden und/oder tamponierenden Ballonkörpern in einem relativ zum Ballon kleineren Lumen entwickelt, in einem transversalen Anschnitt;
- Fig. 15: eine entsprechende, sekretleitende, kanalartige Formation, die sich bei reduziertem Fülldruck tropfenförmig vergrößert, und bei weiterer Reduktion des Fülldrucks U-förmig öffnet, in einer der Fig. 14 entsprechenden Ansicht;
- Fig. 16: einen Trachealtuben-Cuff, wobei die kanalartigen Formationen den Ballonkörper von einer Stirnseite zu der anderen überbrücken, in einer schematischen, perspektivischen Darstellung;
- Fig. 17: eine 2-lagige Ausführungsform einer Ballonwandung, wobei die tragende PUR-Lage mit einer wasserdampfdichten Barriere-Lage aus PVDC kombiniert ist, in einer der Fig. 13 entsprechenden Ansicht;
- Fig. 18: eine 3-lagige Ausführungsform einer Ballonwandung, wobei die tragende PUR-Lage mit einer mittigen Barriere-Lage aus PVDC und/oder EVOH und mit einer die elastischen Aufrichtungseigenschaften von PUR dämpfenden PVC-Lage kombiniert ist, in einer der Fig. 13 entsprechenden Darstellung;
- Fig. 19: einen qualitativen Vergleich zwischen zwei aus elastischem PUR ausgeformten Ballontypen, wobei einer der Bautypen mit einer die elastischen Eigenschaften von PUR modifizierenden Lage von PVC kombiniert ist;
- Fig. 20: eine Seitenansicht auf das distale Ende einer erfindungsgemäßen Ausführungsform eines Blasenkatheters vor dessen Einführen in die Harnblase;
- Fig. 21: eine Schnittansicht durch eine Harnblase, in welcher der Katheter aus Fig. 20 in situ intravesikal platziert ist;
- Fig. 22: einen erfindungsgemäßen Trachealtubus mit durchgängig schaftintegrierter Zuleitung, im Verbund mit einer externen, volumen-bzw. druckregulierenden sowie einer flussrichtenden Vorrichtung;
- Fig. 22a: einen Schnitt durch die Fig. 22 entlang der Linie lia - Ila;
- Fig. 23: eine abgewandelte Ausführungsform eines erfindungsgemäßen Trachealtubus mit einem einkammerigen Ballon, der sowohl tracheal dichtet als auch sub-glottisch tamponiert;
- Fig. 23a: eine wiederum abgewandelte Ausführungsform eines erfindungsgemäßen Trachealtubus mit einer zweikammerigen Anordnung und einem tracheal dichtenden und einem sub- bis supraglottisch tamponierenden Ballon;
- Fig. 24: eine Zuleitung zu dem Ballon eines erfindungsgemäßen Trachealtubus mit einem kombinierten Ventil- und Drosselmechanismus;
- Fig. 25: eine beispielhafte Regler- bzw. Reservoirkomponente für einen erfindungsgemäßen Trachealtubus;
- Fig. 25a: eine zu der in Fig. 25 beschriebenen Komponente zugehörige Druck-Volumenkurve;
- Fig. 25b: einen für zweikammerige Systeme ausgelegten Reservoir- bzw.-Reglerballon;
- Fig. 26: einen erfindungsgemäßen, zuleitungsoptimierten Trachealtubus mit einem Sensorelement im Bereich des tracheal dichtenden Ballonsegmentes und einer mit dem Sensor in einem Regelkreis angeordneten, elektromechanischen Reglereinheit;
- Fig. 27: einen weiteren Trachealtubus gemäß einer abgewandelten Ausführungsform der Erfindung mit einem Sensorelement im Bereich des tracheal dichtenden Ballonsegmentes und einer mit dem Sensor in einem Regelkreis angeordneten, elektronischen Reglereinheit; sowie
- Fig. 28: eine trans-ösophageale Sonde mit angeschlossener Reglereinheit.

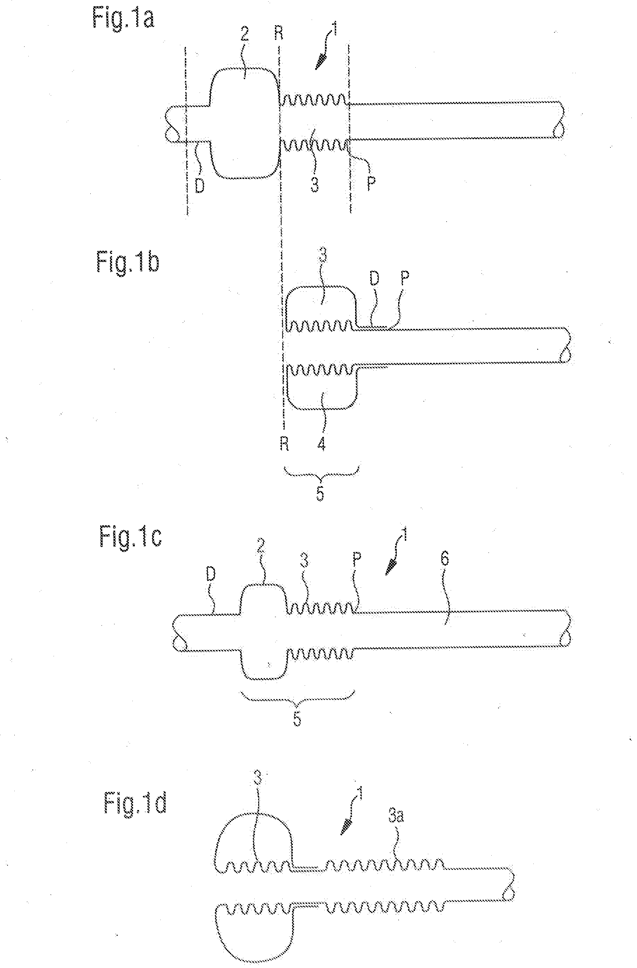

Fig. 1a zeigt beispielhaft in einem schematischen Längsschnitt eine erfindungsgemäß, durch thermische Umformung aus einem durchgängigen Schlauchrohling hergestellte Ballonschlauchfolie 1, die einen sphärisch erweiterten Ballonteil 2, sowie einen, mit einem wellig, ring- oder wendelförmig korrugierten Profil versehenen, den Ballon zum geschlossenen Kompartiment schließenden Schaftschlauchanteil 3 umfasst. Die Rückstülpung der ausgeformten Schlauchfolie erfolgt in der Ebene R. Das distale Ballonende D wird mit dem proximalen Ende P flächig, dicht schließend verbunden. Der wellig ausgeformte Schaftschlauchanteil 3 verleiht der Schlauchfolie innerhalb des geschlossenen Kompartimentes eine radiale Stabilität, die Fülldruckwerten innerhalb des Ballons von bis zu 200 mbar, bevorzugt von 50 bis 100 mbar widersteht bzw. einer nach innen gerichteten, radialen Verformung, Invagination bzw. einem Kollaps der Schlauchfolie entgegenwirkt.

Die Befüllung des Kompartiments erfolgt von außerhalb des Körpers durch eine separate, schlauchförmige Zuleitung, die beispielsweise in den Fügebereich zwischen den Ballonenden D und P verbaut werden kann.

Fig. 1b zeigt eine Fig. 1a entsprechende Ballonschlauchfolie 1 in zu einem befüllbaren Kompartiment 4 geschlossenen Zustand.

Fig. 1c zeigt einen Ballonfolienschlauch wie in Fig. 1a dargestellt, wobei der extrakorporale Anteil der Kathetervorrichtung aus demselben Rohschlauch wie der intrakorporale Anteil der Vorrichtung ausgeformt wird.

Fig. 1d offenbart eine der Fig. 1a entsprechende Ballonschlauchfolie 1 in zu einem befüllbaren Kompartiment 4 geschlossenem Zustand, wobei die wellige Profilgebung der Schlauchfolie über den in der Kavität positionierten, ballontragenden Anteil 3 als trans-luminaler bzw. trans-analer Anteil 3a weitergeführt wird.

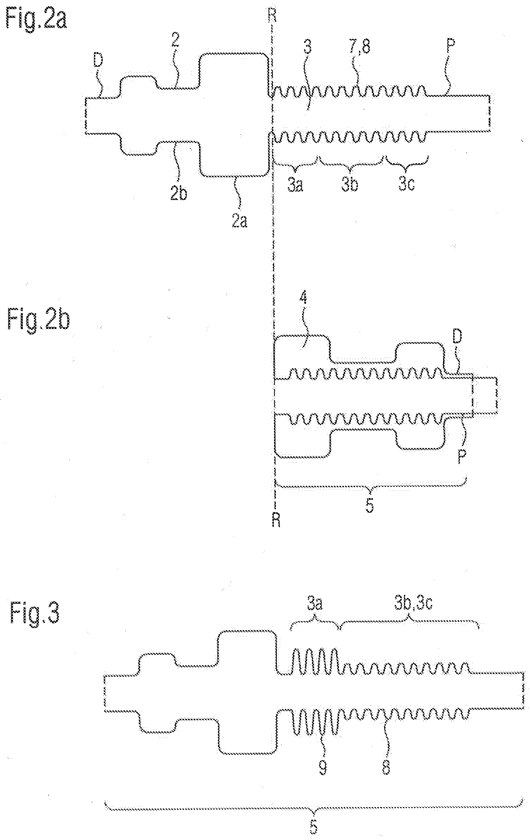

Fig. 2a zeigt eine ausgeformte Schlauchfolie, analog zu Fig. 1a, wobei der Ballonanteil 2 eine spezifische hantelförmige oder optional auch pilzförmige Geometrie aufweist, wobei der Ballonanteil 2 in den Analkanal hinein oder durch diesen hindurchreicht oder den Anus auch optional überragt, und wobei der Ballonanteil über einen wellig profilierten Schaftschlauch 3 rückgestülpt wird, wobei das distale Ballonende D mit dem proximalen Schaftschlauchende P zu einem von außen befüllbaren, dicht schließenden Raum, beispielsweise durch flächige Klebung oder Verschweißung verbunden wird. Sowohl der rektale Anteil 3a als auch der trans-anale Anteil 3b als auch der optionale präanal platzierte Anteil 3c des Schaftschlauches sind mit einem erfindungsgemäßen, ring- oder wendelartigen Wandungsprofil 7 versehen. Die Wandung des Schaftschlauches besteht aus thermoplastischem, ätherbasiertem PUR der Härte nach Shore von 85A bis 90A, sie weist eine Wandungsstärke von 500µm bis 600µm, sowie einen Schaftinnendurchmesser von 15 bis 17mm auf. Der Schaftaußendurchmesser beträgt entsprechend 16 bis 18,2 mm. Ausgehend vom Schaftaußendurchmesser, weist der Schaftschlauch nach außen gerichtete, ring- oder wendelartige, U-förmige Erweiterungen 8 mit einer Amplitude von 2,0 bis 3,0 mm und einem Scheitelabstand von 3,0 bis 5,0 mm auf. Die Breite der U-förmigen Erweiterungen beträgt an der Basis 1,5 bis 3,0 mm. Das wellige Profil 7 erstreckt sich bei dieser Ausführung über eine Länge von insgesamt 70 bis 100 mm.

Bei der kombinierten Ausformung des Schaftschlauch- und des Ballonsegmentes aus einem einzigen, in einem vorausgehenden Schritt extrudierten Rohling, stellen sich bei den beschriebenen, zur Erreichung der erfindungsgemäß geforderten Aufrichtungseigenschaften erforderlichen Schaft- und Wellungsmaße, Ballonwandungsstärken im Bereich des größten rektalen Ballondurchmessers 2a, etwa 55 bis 75mm, von etwa 40 bis 80µm ein. Im mittigen Bereich des Ballons 2b stellt sich bei einem Durchmesser von ca. 25 mm eine Ballonwandungsstärke von ca. 200 bis 300 µm ein.

Fig. 2b zeigt die in Fig 2a dargestellte Schlauchfolie im zum befüllbaren Kompartiment 4 geschlossenen Zustand. In der dargestellten Ausführungsform wird der gesamte intrakorporale Anteil des stuhlableitenden Lumens der Vorrichtung vom Ballon eingehaust.

Fig. 3 zeigt eine weitere Ausführung einer Kopfeinheit 5 in Anlehnung an Fig. 2a, die im Bereich des vom hantelförmigen Ballon umschlossenen Schaftschlauchsegmentes, verschiedenartig ausgeführte, wellig profilierte Anteile umfasst. Die Figur zeigt im Bereich der rektal platzierten Erweiterung 3a eine, relativ zur Wellung im trans-analen Segment 3b, dichter und höher gestellte, U-förmige Ausformungen. Während die Schaft- und Wellungsmaße im trans-analen Segment 3b denen in Fig. 2a entsprechen, weist der Schaftschlauch im distalen Segment 3a konvex gerichtete, U-förmige Erweiterungen 9 von beispielsweise 3,5 bis 5,0 mm Höhe und 2,0 bis 2,5 mm Breite auf. Der Abstand der U-förmigen Erweiterungen 9 zueinander beträgt an der Basis 1,0 bis 2,0 mm. Das höher und dichter gestellte Wellungsprofil stattet den Schaftschlauch in diesem Segment mit einer höheren, lumenerhaltenden Stabilität aus, wodurch einem radialen Kollaps des Segmentes bei rektaler Platzierung bzw. der sich in der jeweiligen Kavität einstellenden Krafteinwirkung auf den Ballon suffizient entgegengewirkt wird bzw. die zum Rektum hin gerichtete, stuhlaufnehmende und drainierende Mündung der Vorrichtung offengehalten werden kann.

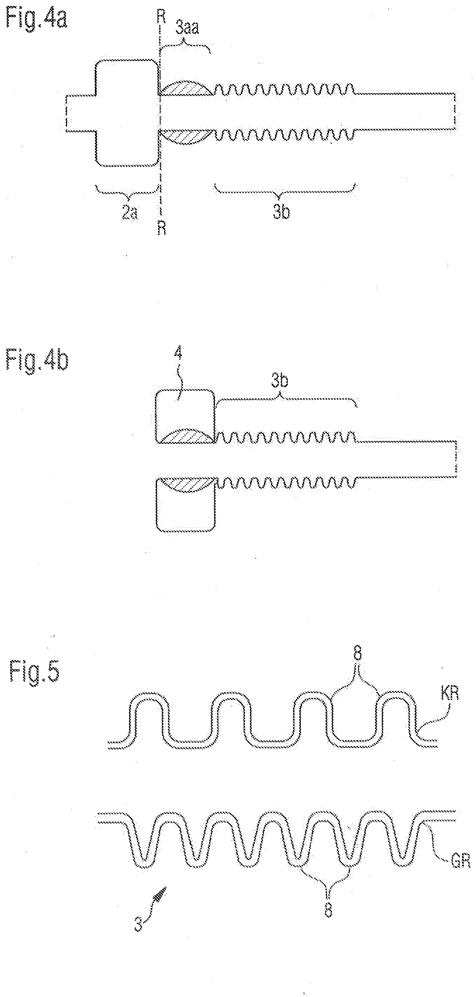

Fig. 4 enthält eine weitere Bauart einer erfindungsgemäß ausgeführten Vorrichtung, wobei der der trans-luminale bzw. trans-anale Anteil 3b des Schaftschlauches durch eine wellige Korrugation der Schlauchwandung in lumenaufrichtender bzw. erhaltender Weise verstärkt wird, das in der Kavität platzierte, den Ballon zum befüllbaren Kompartiment abschließende Schlauchsegment 3a jedoch keine lumenaufrichtende oder -erhaltende Profilgebung aufweist. Der wellungsfreie Abschnitt der zu- und/oder ableitenden katheterartigen Vorrichtung wird bei dieser besonderen Ausführung durch ein separat hergestelltes, das zentrale Lumen stabilisierendes, hülsenartiges, zylindrisches Element 3aa offengehalten. Das Element wird in der bevorzugten Bauweise in den Binnenraum 4 des zurückgestülpten befüllbaren, im Rektum positionierten Kompartiments eingebaut wobei die Innenfläche des Zylinders flächig mit der Außenseite der Ballonfolie, z.B. durch Verklebung mit Lösungsmittel, verbunden ist. Alternativ kann das lumenerhaltende Element auch von distal her in die Mündung des zentralen Kanal des rückgestülpten Folienschlauches eingeschoben und dort fixiert werden.

Fig. 5 zeigt eine weitere beispielhafte Ausführung eines wellig profilierten Schaftschlauches 3, ausgehend von einem inneren Durchmesser D des Schaftschlauches von 12 bis 14 mm. Die Wandung des Schaftschlauches besteht aus einem thermoplastischen, ätherbasierten PUR der Härte nach Shore von 85A bis 90A, sie weist eine Wandungsstärke von 400 µm bis 500 µm auf. Der Schaftaußendurchmesser beträgt entsprechend rund 13 bis 15 mm. Ausgehend vom Schaftaußendurchmesser weist der Schaftschlauch konvex gerichtete, U-förmige Erweiterungen 8 mit einer Amplitude von 1,0 bis 2.0 mm Höhe und 1,5 bis 2,5 mm Breite auf, die im Schaftbereich in einem kleinen Radius KR von ca. 0,25 mm in den Schaftschlauch übergehen Der axiale Abstand der U-förmigen Erweiterungen beträgt an der Basis zum Schaftschlauch 1,5 bis 2,5 mm. Die konvex nach außen gerichteten Erweiterungen 8 können alternativ auch in großen Radien GR von ca. 1,0 mm in den Schaftschlauch übergehen bzw. etwa sinusförmig ausgeprägt sein.

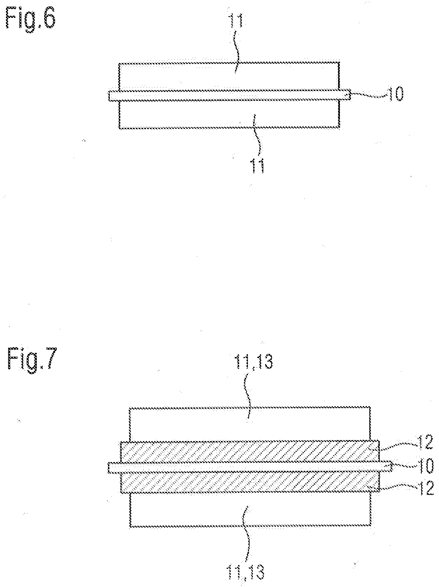

Fig. 6 zeigt einen beispielhaften Lagenaufbau eines ausgeformten Schlauchfolienrohlings 2, umfassend eine mittige Barrierelage 10, bevorzugt aus einem EVOH-basierten Material, beispielsweise des Typs Eval des Herstellers Kuraray, Japan, in einer Lagenstärke von 5 bis 50µm, sowie sich daran beidseits anschließende Lagen 11 eines Trägermaterials, bevorzugt aus PUR, beispielsweise des Typs Elastollan 1185A des Herstellers BASF, mit einer jeweiligen Lagenstärke von beispielsweise 20 bis 100µm.

Fig. 7 zeigt einen alternativen Folienschlauchaufbau, wobei die innere, 5 bis 50 µm starke Barriere-Lage 10, vorzugsweise aus EVOH, beidseits durch eine Polyamidlage (PA) 12 eingefasst ist, welche eine Wandstärke von 10 bis 50 µm aufweist. EVOH und PA ermöglichen ein gut haftendes Koextrusionsverhalten. An die PA-Lagen 12 schließen sich dann wiederum, ein- oder beidseitig, PUR-Lagen 11 oder PVC-Lagen 13 an, welche die Gesamtwandungsstärke auf etwa 50 bis 500 µm ergänzen. Der Vorteil dieser Kombination besteht zum einen in der erhöhten Festigkeit durch den PA-Anteil, zum anderen in den gut kompatiblen Koextrusionseigenschaften der eingesetzten Materialien, die den Einsatz von zusätzlichen Kleberlagen (Tie-Layer) gegebenenfalls erübrigen. Um den Folienschlauch mit elastischen Aufrichtungseigenschaften zu versehen, kann dieser, bevorzugt auf der Außenseite, mit einer oder mehreren PUR-Lagen ergänzt werden.

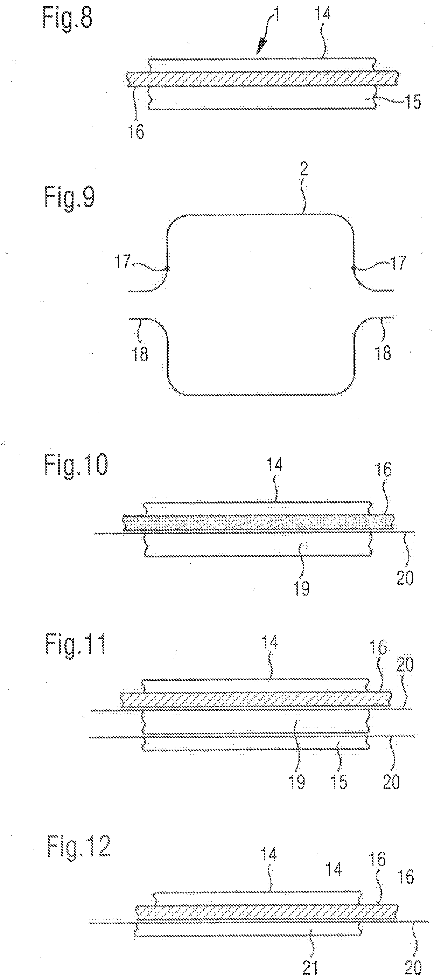

Ähnlich wie Fig. 6, zeigt auch die Fig. 8 zeigt den Wandungsaufbau eines mehrlagig extrudierten Rohschlauchs 1, dessen Wandung aus drei Materiallagen aufgebaut ist, wobei die Außenlage 14 und die Innenlage 15 aus einem thermoplastischen Polyurethan (TPU), oder einem polyurethananteiligen oder -basierten Material bestehen. Insbesondere wird ein TPU eines ester- oder ätherbasierten Typs, bevorzugt mit einer Shore-Härte von 85A bis 95A, empfohlen. Die Gesamt-Lagenstärke der beiden TPU-Lagen 14 und 15 kann dabei in dem Rohschlauch in einem Bereich von 80 µm bis 200 µm liegen, bevorzugt in einem Bereich von 100 µm bis 180 µm.

Dagegen besteht die Zwischenlage 16 aus einem Barrierematerial, vorzugsweise des Typs EVOH, welches besonders effiziente Barriere-Eigenschaften in Bezug auf die Vermeidung des Durchtritts bzw. Übertritts von gasförmigen Luftbestandteilen und Wassermolekülen von der einen auf die andere Seite der mehrlagigen Folienkombination aufweist. Bestimmte EVOH-Typen, wie sie beispielweise vom Hersteller Kuraray Co., Ltd. angeboten werden, gestatten bei der kombinierten, mehrlagigen Extrusion mit Polyurethan den Verzicht auf die Verwendung von haftungsvermittelnden Materiallagen, sogenannten "Tie-Layern", die bei der Kombination verschiedener Materialtypen ansonsten üblicherweise für die Festigung der Verbindung der Einzellagen verwendet werden. Die Gesamt-Lagenstärke der Barrierelage 16 des Rohschlauchs kann dabei in einem Bereich von 10 µm bis 60 µm liegen, bevorzugt in einem Bereich von 20 µm bis 40 µm.

Die Extrusion des Rohschlauches beschränkt sich also in für die Herstellung sehr vorteilhafter Weise von erforderlichen fünf auf nur noch drei Lagen. Die Extrusion kleinerer Rochschlauchmesser von beispielsweise 3 bis 10 mm, bevorzugt von 3 bis 7 mm, wird durch den Wegfall der beiden Tie-Layer somit entscheidend.

Durch den anschließenden Blasform-Schritt werden bei der radialen Dehnung des Schlauchs die Stärken der verschiedenen Schlauchlagen gegenüber dem Rohschlauch reduziert. Dabei findet beispielsweise eine Reduktion der Wandungsstärke der Barrierelage 16 auf einen Bereich von 3 µm bis 10 µm statt, bevorzugt auf einen Bereich von 4 µm bis 8 µm. Gleichzeitig führt die Reduktion der Trägerlagen 14, 15 auf eine Gesamtstärke dieser Lagen 14, 15 von beispielsweise 7 µm bis 20 µm, bevorzugt auf eine Gesamtstärke dieser Lagen 14, 15 von beispielsweise 12 µm bis 15 µm. Daraus resultiert eine Gesamtstärke der fertigen Ballonstruktur zwischen 10 µm bis 30 µm. Im Sinne des Erhalts der Weichfolieneigenschaften in der jeweils ausgeformten ballon- oder schlauchartigen Struktur wird eine Bemessung der anteiligen GesamtWandungsstärken der Barrierelage 16 derart angestrebt, dass der Anteil der Gesamtwandstärke der oder ggf. aller Barrierelagen 16 an der Gesamtwandstärke der ausgeformten ballon- oder schlauchartigen Struktur 2 zwischen einem Drittel und einem Achtel liegt, bevorzugt zwischen einem Viertel und einem Siebtel, insbesondere zwischen einem Fünftel und einem Sechstel.

In entsprechender Weise kann auch PVDC in mittig positionierter Weise zwischen zwei Polyurethan-Lagen angeordnet werden. Die "unvermittelte", direkte Haftung von PVDC an PUR ist jedoch nicht möglich, wodurch eine zusätzliche Lage Haftungsvermittler erforderlich ist. PVDC kann im Gegensatz zu EVOH, welches sich im wässrigen Milieu löst, jedoch direkt einer wässrigen Umgebung exponiert werden, wodurch sich die Anzahl der Materiallagen wiederum auf insgesamt drei, technisch ausführbare Lagen beschränken lässt.

Um bei der Montage des ausgeformten Ballonkorpus auf einem den Ballon tragenden Schaftelement die Option einer dauerhaften Fügung bzw: Klebung mit Lösungsmittel zu gewährleisten, ist die innere, zum ballontragenden Schaftelement hin gerichtete Polyurethanlage 15 der Ballonschlauchfolie 1 vorzugsweise dickwandiger als die äußere PUR-Lage 14.

Fig. 9 beschreibt einen, aus dem Rohmaterial von Fig. 8 bestehenden Ballonkorpus 2. Bedingt durch die mittige Barrierelage 16 aus EVOH oder PVDC und deren flächige, feste Verbindung mit den angrenzende Trägerlagen 14, 15 aus PUR, kommt es bei der elastischen Verformung des dem EVOH bzw. dem PVDC beidseitig anliegenden elastischen Trägermaterials Polyurethan zu einer korrespondierenden Verformung des sich im Gegensatz zum PUR plastisch verformenden EVOH bzw. PVDC. Die Wandungsstärke des EVOH- bzw. PVDC-Anteils verdünnt sich also bei der radialen Ausformung eines Ballonkorpus nicht in linearer Weise vom axialen Zentrum des Rohlings bzw. des Ballons zu dessen größten Durchmesser, wie dies bei einer radialen Streckung eines rein plastischen Materials zu erwarten wäre, sondern die sich effektiv einstellende Ausdünnung entspricht der des sich elastisch verformenden Träger- bzw. Verbundmaterials. In den Bereichen zwischen den Umkehrpunkten 17 der Ballon-Schulterradien, wo die Längskrümmung des Ballons 2 von konkav in konvex übergeht, werden somit in etwa gleichförmige Lagendicken der EVOH- oder PVDC-basierten Barriere-Lagen ermöglicht, wie sich diese auch bei den die mittige Trennlage sandwichartig einschließenden PUR-Lagen einstellt. Eine punktuelle, linienförmige oder flächige Schwächung der Barriere-Funktion der Ballonhülle kann somit weitgehend ausgeschlossen werden.

Um dies zu gewährleisten, sollte das Verhältnis zwischen dem minimalen Durchmesser im Bereich der Schaftenden 18 jenseits der beiden Umkehrpunkte 17 einerseits zu dem maximalen Durchmesser des Ballons 2 zwischen den beiden Umkehrpunkten 17 andererseits möglichst nicht größer sein als 1 : 8, besser wäre ein derartiges Verhältnis von höchstens 1 : 5.

In dem beispielhaften Fall eines Trachealtubus könnte der Gesamtdurchmesser des Rohschlauchs vorzugsweise bei 4 bis 10 mm liegen, beispielsweise bei etwa 7 mm, bei einer bevorzugten Gesamt-Wandstärke des Rohschlauchs zwischen 80 µm und 180 µm. Davon würden 20 µm bis 30 µm auf die Barrierelage 16 entfallen. Während in den nicht verformten Ballonenden diese Stärken auch bei der ausgeformten Ballonstruktur näherungsweise erhalten bleiben, würde in dem Bereich des Ballons 2 eine radiale Aufdehnung in einem Verhältnis von etwa 1 : 5 oder weniger im Verbund mit einer für den Formungsvorgang technisch erforderlichen axialen Streckung von weniger als 1 : 1,5 stattfinden, und dort könnte die Gesamtwandstärke des fertigen Produktes in einem Bereich von 10 µm bis 25 µm liegen, wovon etwa 3 µm bis 5 µm auf die Wandstärke der Barrierelage 16 entfallen.

In dem beispielhaften Fall eines Blasenkatheters könnte der Gesamtdurchmesser des Rohschlauchs vorzugsweise bei 3 bis 5 mm liegen, beispielsweise bei etwa 4 mm, bei einer bevorzugten Gesamt-Wandstärke des Rohschlauchs zwischen 70 µm und 140 µm. Davon würden 20 µm bis 35 µm auf die Barrierelage entfallen. Während in den nicht verformten Ballonenden diese Stärken auch bei der ausgeformten Ballonstruktur näherungsweise erhalten bleiben, würde in dem Bereich des Ballons 2 eine Aufdehnung in einem Verhältnis von etwa 1 : 7 oder weniger stattfinden, und dort könnte die Gesamtwandstärke des fertigen Produktes in einem Bereich von 10 µm bis 20 µm liegen, wovon etwa 3 µm bis 5 µm auf die Wandstärke der Barrierelage 16 entfallen.

Fig. 10 zeigt einen besonderen konzeptionellen Wandungsaufbau, wobei die Innenlage des Rohschlauches 1 bzw. des daraus ausgeformten Ballonkorpus 2 aus einem TPE-basierten Material 19 besteht. Die Wandung besteht in dieser Ausführung also aus der Lagenkombination (von außen nach innen): PUR-EVOH-Vermittler-TPE, mit der Vermittlerschicht 20. Die spezifische Kombination gewährleistet, dass durch die TPE-Innenlage 19 eine technisch einfache Verbindung mit einer auf TPE basierenden, den Ballon 2 aufnehmenden Trägerkomponente hergestellt werden kann. Ferner kann ein so hergestellter Ballon 2 mit für TPE typischen Wasserdampf-Barriere-Eigenschaften ausgestattet werden.

In ähnlicher Weise wie bei Fig. 10 erläutert, nutzt die Erfindung die besonderen elastischen, gleichförmigen Verformungseigenschaften des Polyurethans, wobei sich bei der radialen Expansion der Ballonhülle 2 in dem Segment zwischen den Umkehrpunkten 17 der Schulterradien relativ konstante Wandstärken einstellen. Die PUR-Lage 14 wirkt somit als Träger für die Ausformung der nicht elastischen EVOH-Lage 16 sowie der elastischen TPE-Lage 19. Im Gegensatz zur Blasformung von PUR, können bei der Blasformung von TPE keine Ballonkörper 2 mit einer gleichförmigen, besonders niedrigen Wandungsstärke hergestellt werden. Die Option zur formstabilen Ausdünnung des PUR-Anteils auf sehr niedrige Lagenstärken ermöglicht es, den relativen Anteil der TPE-Lage 19 an der Gesamtwandung zu vergrößern bzw. die spezifischen Dampfbarriere-Eigenschaften des TPE hervorzuheben.

Fig. 11 zeigt einen die Struktur gemäß Fig. 10 ergänzenden, 4-lagigen Aufbau, wobei sich an die innere TPE-Lage 19 eine weitere Lage 15 aus PUR anschließt, ggf. nach einer weiteren Vermittlerschicht 20. Es wird somit die Option der Lösungsmittel-Verklebung auf einer den Ballon 2 tragenden Schaftkomponente integriert, sowie die beiden Mittel-Lagen Barrierelage 16 und TPE-Lage 19 beidseits vom stützend ausformenden Trägermaterial 14, 15 sandwichartig eingeschlossen. Der Wandungsaufbau entspricht also (von außen nach innen): PUR-EVOH-Vermittler-TPE-Vermittler-PUR, oder PUR-Vermittler-EVOH-Vermittler-TPE-Vermittler-PUR. Die Lagenstärken im ausgeformten Ballon 2 verteilen sich dabei beispielhaft wie folgt: PUR (5-7µm) - EVOH (1-5µm) - Vermittler (1-3µm) - TPE (5-15µm) - Vermittler (1-3µm) - Vermittler (1-3µm).

Fig. 12 zeigt einen Ballonkörper, bestehend aus einer äußeren Lage 14 eines thermoplastischen Polyurethans mit einer sich nach innen direkt, ohne Haftungsvermittler anschließenden Barriere-Lage 16 aus EVOH und einer sich daran anschließenden Lage aus Vermittler 20 und PVDC 21. Die Lagenstärken im ausgeformten Ballon verteilen sich dabei beispielhaft wie folgt: PUR (5-7µm) - EVOH (1-5µm) - Vermittler (1-3µm) - PVDC (1-5µm).

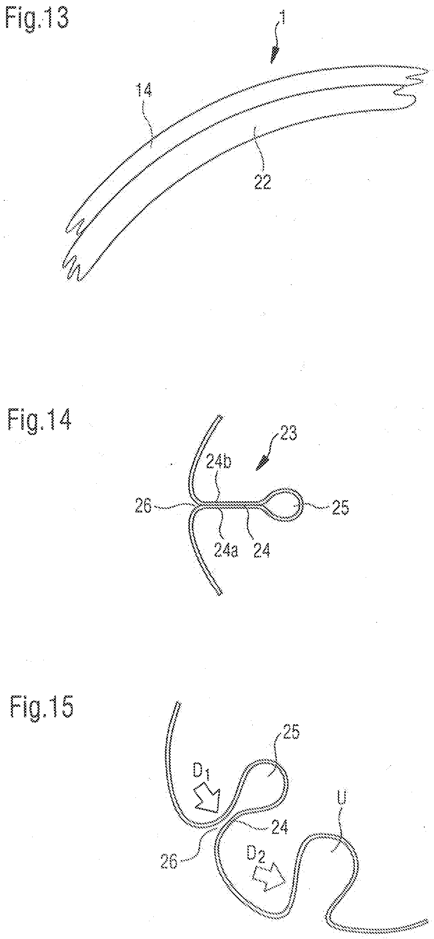

Fig. 13 zeigt in schematischer Ausführung einen beispielhaften, erfindungsgemäßen, 2-lagigen Aufbau einer Ballonschlauchfolie 1, wobei vorzugsweise die äußere, dem jeweiligen Lumen oder Hohlraum zugewandte Materiallage 14 aus thermoplastischem PUR des Typs Elastollan 1100 mit einer Härte nach Shore von 90A besteht und eine anteilige Wandungsstärke von 5 bis 10 Mikrometern aufweist. Bevorzugt besteht die dem Binnenraum des Ballons 2 zugewandte Materiallage 22 aus einem PVC mit einer Härte nach Shore von 70A und weist eine anteilige Wandungsstärke von 15 bis 20 Mikrometern aus. Die beiden Polymere werden vorzugsweise direkt, also ohne eine haftungsvermittelnde Zwischenlage, durch einen Koextrusionsprozess, in fest aneinander haftender Weise, flächig verbunden hergestellt. Die 15 bis 20 Mikrometer starke PVC-Lage 22 wirkt zum einen der elastischen Aufrichtung der zu einer ösenartigen Formation gefalteten anteiligen PUR-Lage 14 in dämpfender, die Geschwindigkeit und das Ausmaß der Aufrichtung reduzierender Weise entgegen. Zum anderem reduziert die anteilige PVC-Lage 22 den Durchtritt bzw. die Migration polarer Substanzen durch die beschriebene PUR/PVC-Lagenkombination, und reduziert somit unerwünschte Kondensations- und Akkumulationseffekte von Flüssigkeit im Binnenraum des Ballons 2, insbesondere von Wasser.

Im Rahmen der Erfindung können die aus pur und PVC bestehenden Wandungslagen 14, 22 auch derart innerhalb des Lagenverbundes angeordnet werden, dass sich sie PVC-Lage auf der Ballon-Außenseite befindet, die dann von einer inneren PUR-Lage 15 begleitet wird.

Neben 2-lagigen Ballonwandungen sind auch 3-lagige Ausführungsformen möglich, wobei bspw. eine PUR-Lage 14 zwischen zwei PVC-Lagen 22 in sandwichartiger Weise eingefasst sein kann. Bei einer Gesamtwandungsstärke von 30 Mikrometern kann die Verteilung der Einzellagen beispielsweise außen 12 µm PVC, mittig 6 µm PUR und außen 12 µm PVC aufweisen. Diese Ausführungsform ist besonders vorteilhaft bei der Begrenzung unerwünschter Migrationseffekte polarer Substanzen, wie zum Beispiel Wasser.

Fig. 14 zeigt im Schema den transversalen Anschnitt einer sekretleitenden Invagination 23, wie sie sich bei einem residual, d.h. auf ein Übermaß ausgeformten, dichtenden und/oder tamponierenden Ballonkörper 2 bei der Platzierung innerhalb eines in Relation zu dem residual bemessenen Ballon 2 kleineren Lumens oder Raums durch Einstülpung der überschüssigen Ballonwandung 1 faltenartige Strukturen entwickeln. Insbesondere bei zyklisch wechselnden Fülldruckwerten innerhalb des Ballons 2 kommt es im Anwendungsverlauf zu einer typischen, vom Umfang zum Zentrum des Ballons 2 gerichteten, radspeichenartigen Anordnung derartiger Einstülpungen 23.

Die Invaginationen 23 weisen dabei jeweils einen stegartigen, flächig geschlossenen Anteil 24 auf, während sich am blinden, zum Ballonzentrum gerichteten Ende jeder Invagination eine ösenartige Formation 25 ausbildet. Im Bereich der sich formierenden Öse schlägt die Wandung 1 der Ballonhülle 2 um 180 Grad um, wobei durch die elastischen Aufrichtungseigenschaften der in die Wandung integrierten PUR-Lage eine ausgeprägte, öffnende Wirkung auf die ösenartige Formation generiert wird. Abhängig von der Größe der Querschnittsfläche der jeweiligen ösenartigen Formation, sowie von einer größtmöglichen Vermeidung bzw. Reduktion von zyklischen Kalibersprüngen der Öse, resultiert die jeweilige, zu einem bestimmten Zeitpunkt effektive Dichtungswirkung des Ballons. Kleine Querschnittflächen der Öse wirken sich aufgrund von Kapillareffekten auf Sekrete, die sich innerhalb der Öse befinden, in flusshemmender Weise bis hin zur vollständigen Stase des Sekretes bzw. des Öseninhaltes aus. Der den freien Durchfluss von Sekret hemmende Effekt geht mit zunehmender Weitung bzw. Vergrößerung der Querschnittfläche der Öse verloren.

Die dichtungsrelevante Querschnittsfläche der ösenartigen Formation 25 wird neben der jeweiligen Eigenschaft zur elastischen Aufrichtung der ösenartig umgeschlagenen Ballonwandung durch den jeweils aktuell im Ballon herrschenden Fülldruck bestimmt, der insbesondere den beiden Wandungslagen 24a und 24b des stegartigen Anteils 24 der Invagination 23 anliegt, und diese flächig, in dicht schließender Weise aneinander presst, wobei im Bereich des Umschlags der beiden Wandungslagen, d.h. an dem blinden Ende der betreffenden Invagination, ein offenes Lumen verbleibt.

Die Gesamtwandungsstärke eines erfindungsgemäß ausgeführten Ballons 2 soll vorzugsweise 30 µm nicht überschreiten. Bei der bevorzugten Ausführung des Ballons 2 liegt das Verhältnis der anteiligen Wandstärke der PUR-Lage zur anteiligen Wandstärke der PVC-Lage zwischen 1 : 2 und 1 : 4, bevorzugt bei einem Verhältnis von 1 : 3.

Kommt es beispielsweise bei einer spezifischen, wie in Fig. 13 beschriebenen Kombination von Lagen 14, 22 zu einer zyklischen, durch die Eigenatmung eines Patienten generierten Schwankung des Fülldrucks in dem Ballon 2 eines Trachealtubus zwischen 30 und 5 mbar, so resultiert dies in einem Zuwachs der die Dichtungseffizienz des Ballons 2 bestimmenden Querschnittfläche der Ösen 25 von 10% bis 25%, in der Regel jedoch von nicht mehr als 20%. Die größten, innerhalb einer jeweiligen ösenartigen Formation gemessenen Ösendurchmesser erfindungsgemäß hergestellter Ballons 2 liegen bei einem durchgängigen Fülldruck von 30 mbar bei ca. 30 bis 120 µm, bevorzugt bei ca. 40 bis 80 µm.

Bei zyklischen Schwankungen des Fülldrucks im Ballon 2 von beispielsweise 20 Wechseln pro Minute und Druckamplituden oder Druckextremwerten zwischen 30 und 5 mbar, bleiben die Dichtungseigenschaften des erfindungsgemäßen Ballons 2, zum Beispiel bei der konkreten Verwendung für die tracheale Sekretdichtung, weitestgehend erhalten. Pumpartige, der Eigenatmung des Patienten synchron folgende, zyklisch melkende Effekte auf die ösenartige Formation 25 bzw. auf die sich aus den Ösen 25 ausformenden Kanäle, wie diese in der medizinischen Literatur bei dickwandigen, einlagigen, PVC-basierten Cuffs der Wandungsstäre von 70 bis 120 Mikrometer beschrieben sind, bleiben bei einem erfindungsgemäß ausgeführten Trachealtuben-Cuff weitgehend aus.

Fig. 15 zeigt eine der Fig. 14 entsprechende, ösenartige Formation 25 im Zustand einer relativ zu Fig. 14 reduzierten Fülldrucksituation. Unterschreitet der Fülldruck einen gewissen dichtungskritischen Fülldruck D1, beginnt sich der Eintrittsbereich 26 an der Basis der Invagination 23 zu öffnen, und die ösenartige Formation 25 weitet und verlängert sich, vom blinden, inneren Ende der Invagination beginnend, zur äußeren Basis 26 der Invagination 23 hin fortschreitend. Das stegartige, dicht schließende Segment 24 der Invagination 23 verkürzt sich entsprechend. Bei einem weiteren Abfall des Fülldrucks auf einen Wert D2 öffnet sich das stegartige Segment 24 vollständig, und die Invagination 23 geht in eine flächig offene U-Form U über.

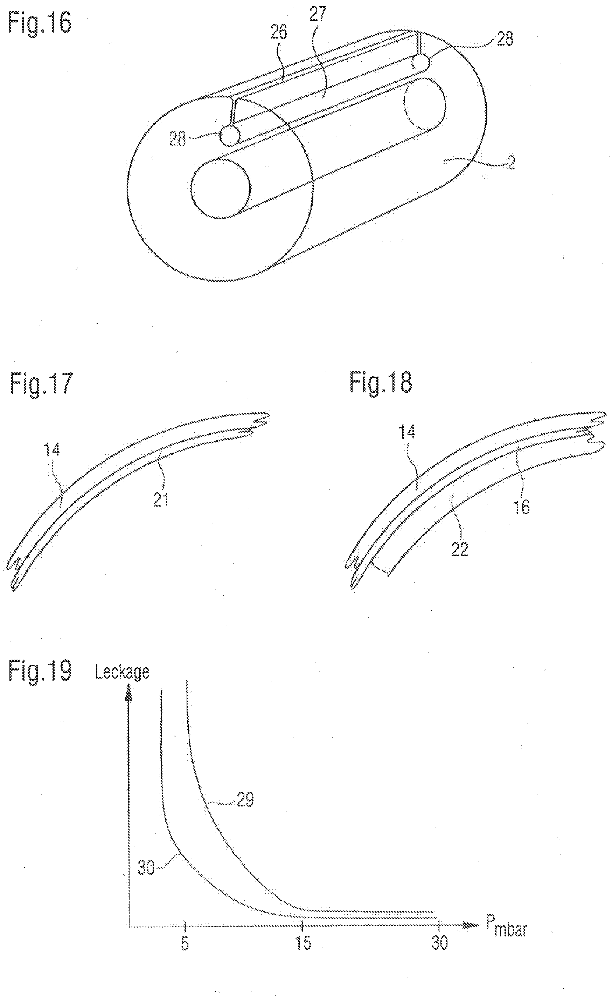

Fig. 16 zeigt schematisch, am Beispiel eines zylindrischen Dichtungsballons 2, wie dieser beispielsweise als sekretdichtender Trachealtuben-Cuff zur Anwendung kommt, kanalartige Formationen 27, die aus den ösenartigen Umschlagformationen 25 am blinden Ende der jeweiligen Invaginationen 23 hervorgehen. Die kanalartigen Formationen 27 erstrecken sich dabei von einer Stirnseite 28 des Ballonzylinders 2 zur gegenüberliegenden Stirnseite 28 in durchgängiger Weise, bzw. nehmen bei kontinuierlicher Belastung mit zyklisch wechselnden Fülldrucken in vielen Fällen eine in etwa parallele Ausrichtung zur Zylinderachse des Ballons an, und ermöglichen somit die Leckage von Flüssigkeiten oder Sekreten von der einen zu der anderen Stirnseite des ein Lumen oder einen Binnenraum eines Patienten in dichtender Weise verschließenden oder raumfüllend tamponierenden Ballons.

Fig. 17 zeigt eine besondere, 2-lagig ausgeführte Ballonwandung 1, wobei die den Ballon 2 stabilisierende PUR-Lage 14 mit einer wasserdampf- und gasdichten Barriere-Lage 21 aus PVDC kombiniert ist. Die PVDC-Lage 21 kann sowohl zur Außenseite des Ballons 2 als auch zu dessen Innenseite hin orientiert sein. PVDC wirkt bereits in sehr dünnen Lagenstärken sehr effizient wasser- und gasdichtend. Die vorgeschlagene Kombination bietet somit die Grundlage für die Herstellung besonders vorteilhafter, niedriger Gesamtwandungsstärken des Ballons im Bereich von 10 bis 15 Mikrometern, wie sie im Sinne einer möglichst kleinen bzw. möglichst konstanten, nicht alternierenden Querschnittsfläche der ösenartigen Formation 25 vorteilhaft sind. Die PUR-Lage 14 weist dabei eine Lagenstärke von beispielsweise 5 Mikrometer auf, die PVDC-Lage 21 dagegen eine Stärke von beispielsweise 5 bis 10 Mikrometer.

Fig. 18 zeigt eine besondere, 3-lagige Ausführungsform einer Ballonwandung, wobei die elastische PUR-Lage 14 mit einer mittig angeordneten, gas- und wasserdampfdichte Barriere-Lage 16, beispielsweise aus PVDC oder alternativ auch aus EVOH, und einer die elastischen Aufrichtungseigenschaften von PUR erfindungsgemäß dämpfenden Lage 22, bevorzugt aus PVC niedriger Shorehärte, kombiniert ist. Bei einer Gesamtwandungsstärke von beispielsweise 25 Mikrometer weist die PUR-Lage 14 dabei eine Lagenstärke von beispielsweise 5 Mikrometern auf, die gas- und wasserdampfdichte Barriere-Lage Lage 16 aus bspw. PVDC eine Stärke von 5 Mikrometer, und die dämpfende Lage 22 aus bspw. PVC eine anteilige Lagenstärke von 15 Mikrometer.

Fig. 19 veranschaulicht anhand zweier Graphen 29, 30 in qualitativer Weise, wie sich die im Binnenraum des Ballons 2 herrschenden Fülldrucke auf die für die Dichtungseffizienz der jeweiligen, dichtenden oder tamponierenden Katheter- oder Device-Anwendung relevante Querschnittfläche der ösenartigen Formation 25 auswirken. In einer vergleichenden Näherung wird gemäß einem Graph 29 ein residual bemessener, radiale Invaginationen der residualen Ballonhülle ausformender, einlagig aus PUR hergestellter Ballon 2 mit einer Wandungsstärke von 15 Mikrometer, gefertigt aus Material des Typs "Elastollan 1190A", einem Graph 30 entsprechend einem residual ausgeformten und bemessenen Ballon 2 aus zweilagigem Material, bestehend aus einer erfindungsgemäßen Kombination einer PUR-Lage 14 und einer PVC-Lage 22, mit einer Gesamtwandungsstärke von 20 Mikrometer, wie in Fig. 13 in beispielhafter Weise für die Technologie beschrieben, gegenübergestellt. Bei ca. 20 zyklischen Schwankungen pro Minute, die jeweils den Druckbereich zwischen 30 mbar bis 15 mbar durchlaufen, weisen beide Ballontypen einen vergleichbar effizienten Dichtungseffekt auf, der einer nahezu vollständigen Dichtung entspricht. Reichen die unteren Werte der Druckschwankungen jedoch in den Bereich von 15 bis 5 mbar, trennen sich die beiden Graphen 29, 30, wobei sich die Querschnittsfläche der Öse 25 bei der Variante 29 im Vergleich zur Variante 30 des Ballons 2 gemäß Fig. 16 um ca. 10 bis 25% vergrößert. Bei dem einlagigen Ballon gemäß Graph 29 geht die Dichtung in einem Druckbereich unterhalb von 5 mbar gänzlich verloren, was bei dem erfindungsgemäßen, mehrlagigen Ballon 2 aus einer erfindungsgemäßen Kombination einer PUR-Lage 14 mit einer PVC-Lage 22 erst unterhalb von etwa 3 mbar der Fall ist.

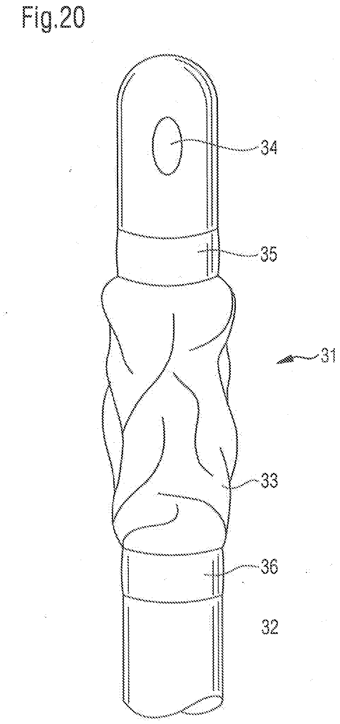

Fig. 20 zeigt das distale Ende eines Hamblasenverweilkatheters 31 in der Seitenansicht. Der Katheter 31 ist mit einem Schaft 32 und einem am Schaft 32 befestigten Ballon 33 versehen. Proximal zum distalen Ende des Schafts 32 befindet sich eine Öffnung 34, die dazu dient, Urin oder andere Flüssigkeiten durch das Innere des Katheters 31 abzulassen. Der Ballon 33 ist in einem Grundzustand dargestellt, d. h. im Ruhezustand und vollständig kollabiert. Durch die Enden des Ballons 33 werden Bänder 35 und 36 gebildet, die als fluiddichte Verbindungen zwischen dem Schaft 32 und dem Ballon 33 dienen. Die Verbindung der Bänder 35, 36 mit dem Schaft 32 selbst kann durch einen geeigneten Klebstoff, durch Ultraschallschweißen oder durch eine andere Verbindungstechnik erfolgen.

Der Ballon 33 liegt im entleerten oder zusammengelegten Zustand eng an der Wand der Schaftes 32 an, wobei sich die folienartige Struktur des Ballons 33 zufällig oder in vorkonfigurierten Mustern faltet. Der Ballon 33 ist mit zwei Schlauchenden 35, 36 bzw. Schaftbefestigungsstücken versehen, die an dem Katheterschaft 32 befestigt sind.

Der Schaft 32 des Katheters 31 besteht kann aus einem elastischen Material bestehen, und kann bevorzugt aus PUR oder PVC gefertigt sein. Ebenfalls kommen in Frage LDPE. LLDPE, SEBS, Silikon oder Naturkautschuk. Der Katheterschaft 32 kann bevorzugt einen drei- oder auch zwei-lumigen Aufbau zeigen.

Bei einer günstigen Materialwahl ist eine Schaftwandstärke von etwa 0,4 bis etwa 0,8 mm, vorzugsweise von etwa 0,4 bis etwa 0,6 mm, ausreichend. Der Katheterschaft 32 soll seine Steifigkeit bzw. Knicksicherheit behalten, wie sie für das Einführen in die Harnröhre bei Patientenanwendungen erforderlich ist.

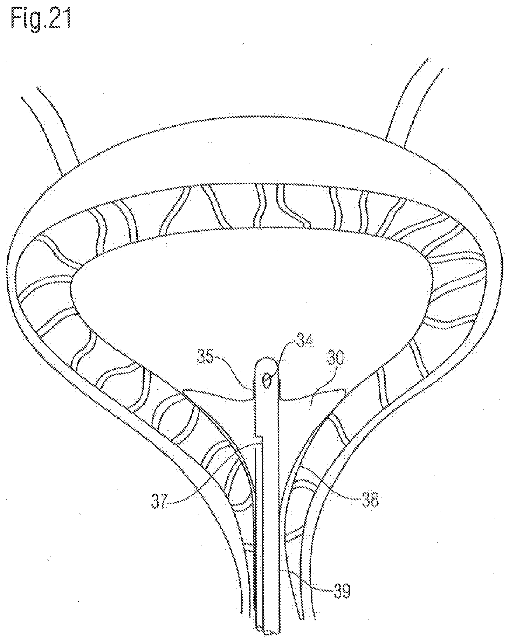

Wie in FIG. 21 gezeigt, ist der Ballon 33 auf sein Arbeitsmaß oder residual ausgeformt und muss daher im Betriebszutand nur so weit aufgeblasen werden, dass sein Volumen nur teilweise gefüllt ist, so dass der Druck in dem Ballon etwa bei dem Umgebungsdruck bleiben kann, das heißt, der Druck im Inneren des Ballons 33 ist ungefähr gleich dem Druck an der Außenseite des Ballons. Dies ermöglicht es dem Ballons 33, sich anatomisch in das Trigonum vesicae 38 einzuformen und die innere Harnröhrenöffnung 39 auszufüllen.

Um eine Befüllung des Ballons 33 zu ermöglichen, ist der Katheterschaft 32 in dem vom Ballon 33 überdeckten Bereich mit einer Öffnung 36 oder mehreren solchen Öffnungen versehen. Diese Einfüllöffnungen 37 müssen nicht rund sein und können tatsächlich eine quadratische oder rechteckige Form haben. Es hat sich herausgestellt, dass diese Form im Wesentlichen verhindert, dass der dünne Film des Ballons die Öffnung oder Öffnungen verschließen kann.

Der Ballon 33 wird vorzugsweise wie oben beschrieben in längsgestreckter Form am Schaft 32 befestigt. Das Ruhevolumen der auf diese Weise angelegten Manschette ist typischerweise kleiner als 0.08 ml, bevorzugt im Bereich von nur 0,02 bis 0,04 ml. In vielen der Ausführungsformen können die vorgeformten Ballonelemente ein Arbeitsvolumen von 5 ml und einen Wanddickenbereich von etwa 5 bis etwa 10 Mikrometer haben. Bei diesen speziellen Ausführungsformen mit einem Arbeitsfüllvolumen von 30 ml kann die Wandstärke der Ballonhülle vorzugsweise in den Bereich von etwa 5 bis etwa 15 Mikrometer fallen.

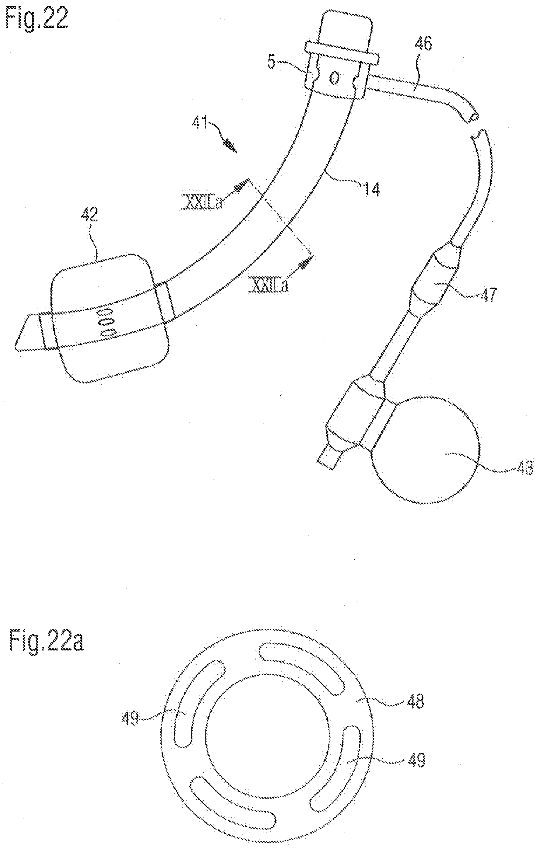

Fig. 22 beschreibt in einer beispielhaften Gesamtübersicht verschiedener funktions-optimierender Komponenten einen trachealen Beatmungskatheter 41 (Trachealtubus) zur dynamischen trachealen Dichtung bei zyklisch alternierenden thorakalen Drucken durch flussoptimierte Verschiebung eines mit Druck beaufschlagten Füllmediums, zwischen einem tracheal positionierten Dichtungsballon 42 (Cuff) und einem extrakorporalen Regler- bzw. Reservoirelement 43.

Formgebung und Dimension der Vorrichtung der erfindungsgemäßen Vorrichtung entsprechen dabei weitgehend einem herkömmlichen Trachealtubus. Der tracheal dichtende Ballon ist am distalen Ende des Tubus mit seinen beiden Enden dicht schließend mit dem ballon-tragenden Katheterschaft verbunden. Der Schaftkorpus 44 weist bevorzugt eine in den Schaft integrierte, großlumige bzw. mehrlumige Zuleitung zum Cuff auf. Am proximalen Schaftende 45 werden die jeweiligen zuleitenden Lumina zusammengeführt und von dort aus mit einer groß-lumigen Zuleitung 46 an das Reglerelement 43 angebunden. Zur Vermeidung von schnellen retrograden Entleerungen zum Reservoir hin, sowie für den verzögerten Druck- bzw. Volumenausgleich zwischen den endständigen Kompartimenten ist in die Zuleitung ein Element 47 mit einer kombinierten Ventil- und Drossel-Funktion integriert. Im Verbund der kommunizierenden Volumina von Ballon 42, Zuleitung 46, Ventil-Drossel-Element 47 sowie Reservoir- bzw. Reglerelement 43 entsteht ein gemeinsamer Binnenraum, in dem ein konstanter, durch das Reservoir bzw. den Regler 43 definierter Druck aufrechterhalten wird. Als bevorzugtes Medium zur Befüllung des kommunizierenden Binnenraumes kommt im Rahmen der Erfindung Luft zur Anwendung.

Die im Rahmen der Erfindung beschriebene Technologie zur möglichst widerstandsminimierten, optimal raschen Volumenverschiebung, bei gleichzeitig möglichst geringen Druckgradienten zwischen einem tracheal positionierten Ballon und einer extrakorporalen Reglereinheit, soll eine druckstabilisierende Volumenkompensationen innerhalb des tracheal dichtenden Ballons ermöglichen, die im optimalen Falle nach nicht mehr als 10 bis 20 Millisekunden nach Einsetzen eines atemmechanisch ausgelösten Druckabfalls abgeschlossen sind.

Fig. 22a zeigt einen beispielhaftes, mehrlumig ausgeführtes Schaftprofil 48 im Querschnitt. Die in die Schaftwandung integrierten Zuleitungs-Lumina 49 sind im Sinne einer möglichst geringen Vergrößerung des Schaftaußendurchmessers bevorzugt flach bzw. mit einer möglichst geringen radialen Höhe ausgeführt.

Als Material für den Schaft 44 kommt bei der in Fig. 22 und 22a gezeigten Basis-Bauweise bevorzugt PVC des Durometerbereiches Shore 80A bis 90A in Frage.

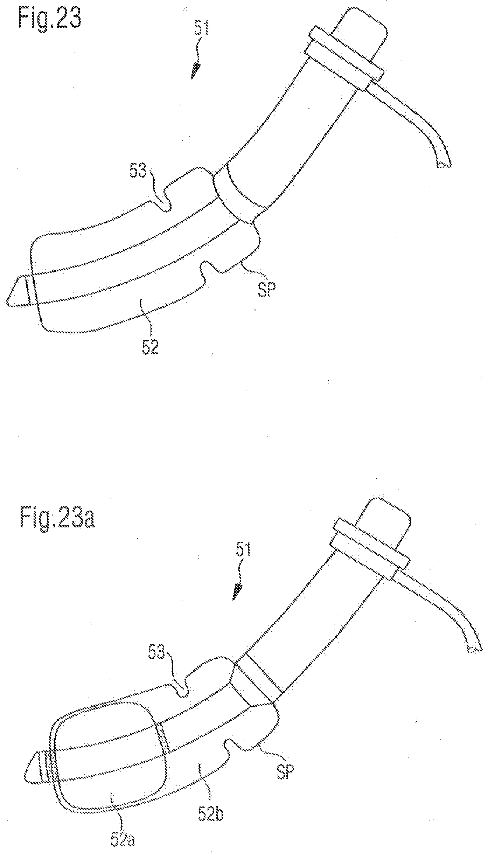

Fig. 23 zeigt einen Trachealtubus 51 mit einem über die Stimmlippenebene hinaus verlängerten Ballonelement 52. Das Ballonelement 52 soll, entsprechend den weiteren im Rahmen der Erfindung beschriebenen Ausführungsformen, vorzugsweise aus ausgeformtem Folienmaterial bestehen, welches im Durchmesser derart residual bemessen ist, dass es zur Dichtung des trachealen Lumens nicht gedehnt werden muss und sich der Schleimhaut des Organs unter Einfaltung der überschüssigen Ballonhülle weitgehend spannungslos anschmiegt. Die Erfindung bevorzugt polyurethanbasierte Ballonfolien, die im Bereich des tracheal dichtenden Ballonsegmentes eine Wandstärke von bevorzugt 5 bis 20 µm, weniger bevorzugt von 20 bis 50 µm aufweisen.

Für das tracheal dichtende Ballonelement kommen erfindungsgemäß bevorzugt Polyurethane der Härten Shore 70A bis 95A bzw. 55D bis 65D zur Verwendung. Besonders bevorzugt kommen Shore-Härten des Bereiches 85A bis 95A zum Einsatz.

Während das Ballonelement im einfachen Falle für die Dichtung im Bereich des Übergangs des unteren zu mittleren trachealen Drittel dimensioniert ist, wie dies bei konventionellen Trachealtuben oder Trachealkanülen üblich ist, kann das tracheal dichtende Ballonsegment im Rahmen der Erfindung auch nach proximal verlängert werden und über die Stimmlippen hinaus in den Bereich des supra-glottischen, unteren Rachens reichen. Der Korpus des Ballonelementes 52 wird dabei bevorzugt zylindrisch ausgeformt. Er kann im Bereich der Stimmlippenebene mit einer zirkulären Verjüngung 53 zur Aufnahme der Stimmlippen versehen sein.

Die nach proximal verlängerte Ausführung des tracheal dichtenden Ballons 52 gestattet ein besonders großes Ballonvolumen, welches in der Lage ist, eine gewisse druckerhaltende Pufferwirkung zu entwickeln, wenn es im trachealen Abschnitt des Ballonkorpus zu atemmechanisch bedingten Vergrößerungen des trachealen Querschnitts bzw. zu einer nachlassenden transmuralen Kraftwirkung auf den tracheal dichtenden Ballon kommt. Reicht das proximale Ballonende aus dem Thorax heraus, ist dieses extra-thorakale Segment der thorakalen Atemmechanik nicht ausgesetzt, was den dämpfenden Effekt der extra-korporalen Volumenreserve entsprechend unterstützt, und die erfindungsgemäße, dynamisch wirkende, dichtungserhaltende Funktion der Vorrichtung weiter verbessert.

Ferner kann durch die große Kontaktfläche eines nach proximal verlängerten, tracheal dichtenden Ballons 52 ein größtmöglicher Migrationsweg für Sekrete bzw. darin enthaltene Erreger ermöglicht werden.

In der Fig. 23a wird ein der Fig. 23 entsprechender Trachealtubus 51 vorgestellt, der zwei ineinander geschachtelte Cuffs 52a, 52b beinhaltet und dadurch neben der trachealen Dichtung durch den inneren Ballon 52a bei relativ höherem Dichtungsdruck auch eine Tamponade des sub-glottischen Raumes durch einen äußeren Ballon 52b bei einem relativ niedrigeren Tamponadedruck ermöglicht. Neben der konzentrischen Anordnung ist auch eine, sequentielle, zwischenraumfreie Anordnung von tracheal dichtendem Cuff (distal) und sub-giottischem Tamponadeballon (proximal) denkbar.

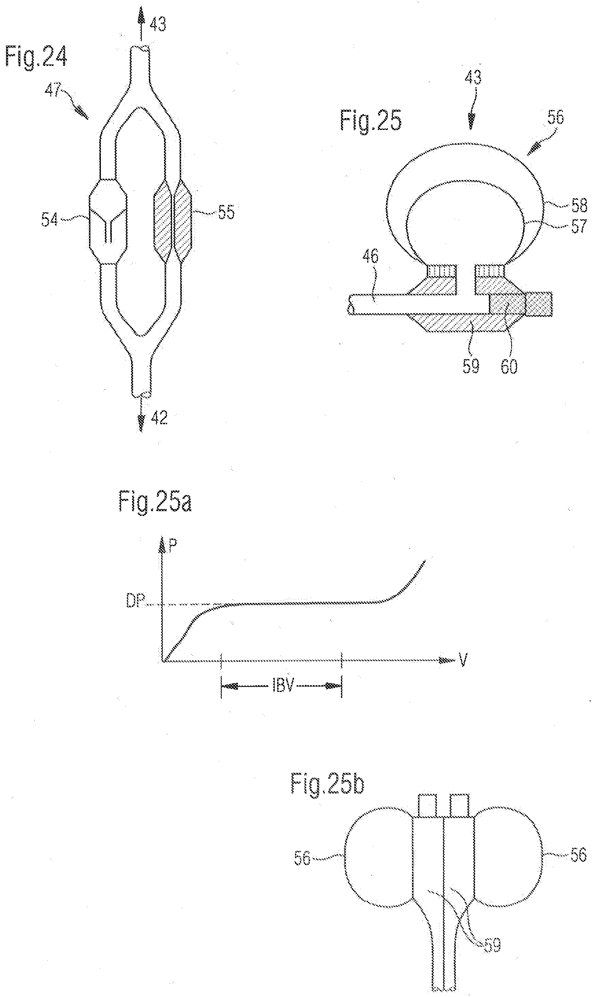

Gemäß Fig. 24 kann, um plötzliche, retrograde, potentiell dichtungskritische Entleerungen des Ballonvolumens zum Regle bzw. zum Reservoir hin zu vermeiden, wie diese beispielsweise beim mehrfachen Husten des Patienten in kurzer Sequenz erfolgen können, kann die verbindende Zuleitung 46 zwischen dem Schaft 44 und der regelnden Reservoireinheit 43 mit einem flussrichtenden Ventil 54 ausgestattet sein, welches den raschen Rückschlag von Füllmedium verhindert. Das Ventil 54 soll dabei derart konstruiert sein, dass es den offenen, anterograden Volumenstrom vom Regler zum Ballon hin möglichst wenig beeinträchtigt.

Um durch das Ventil 54 verursachte Poolungs-Effekte von Medium im Ballon 42 zu vermeiden, wird das Ventil 54 bevorzugt mit einer nicht-flussgerichteten, nach beiden Seiten hin offenen Bypass-Drossel 55 ausgestattet, die einen langsamen, verzögerten Druck- bzw. Volumenausgleich zwischen den beiden endständigen Kompartimenten Ballon 42 und Regler 43 ermöglicht. Mit anderen Worten, die Drossel 55 ist parallel zu dem Rückschlagventil 54 geschalten. Das Rückschlagventil 54 ist derart orientiert, dass es bei einem Differenzdruck von der Reservoir- oder Reglereinheit 43 zu dem Cuff bzw. Dichtungsballon 42 hin öffnet und das Medium schnell von der Reservoir- oder Reglereinheit 43 zu dem Cuff bzw. Dichtungsballon 42 hin strömen lässt. Bei einem umgekehrten Druck schließt das Rückschlagventil 54, und eine Strömung von dem dem Cuff bzw. Dichtungsballon 42 zu der Reservoir- oder Reglereinheit 43 hin kann nur durch das dazu parall geschaltete Drosselelement 55 erfolgen, das jedoch einen geringeren freien Strömungsquerschnitt hat, so dass die Strömungsmenge pro Zeiteinheit in dieser Strömungsrichtung kleiner ist als von der Reservoir- oder Reglereinheit 43 zu dem Cuff bzw. Dichtungsballon 42 hin.

Es wäre Im einfachsten Ausführungsfalle denkbar, die jeweilige, dichtende Ventilfläche mit einer kleinen Bohrung bzw. Öffnung zu versehen, die einen entsprechenden gedrosselten Volumenstrom erlaubt.

Fig. 25 zeigt eine beispielhafte Ausführung einer kombinierten Regler- bzw. Reservoir-Einheit 43. Diese Einheit 43 weist einige konzeptionelle Merkmale eines sogenannten Lanz-Reglers auf. Die wesentliche funktionelle Komponente der Einheit 43 besteht wie bei der Einheit nach Lanz aus einem besonderen volumendehnbaren Ballonblase 56 aus hoch-elastischem Material, welches von einer gewissen vorgeformten, beispielsweise sphärischen Grund- bzw. Ruhefigur 57, durch Befüllung in einen gedehnte Arbeitsfigur 58 übergeht. Bei Verwendung geeigneter Materialien kann ein bestimmtes Expansionsverhalten der Ballonblase erreicht werden, wobei sich das Volumen bei isobarer Druckentwicklung im Ballon 42 vergrößert. Die zugehörige, konzeptionell angestrebte Druck-Volumenkurve ist in Fig. 25a dargestellt. Die Ballonblase 56 besteht vorzugsweise aus einem natürlichen, latex-ähnlichen Material oder aus einem synthetischen Stoff, wie Isoprenverwandten Material. Die Ballonblase 56 sitzt einem Sockelgehäuse 59 auf, in das in bevorzugter Weise ein Einwegeventil 60 für die Befüllung mit Luft eingebaut ist.

Fig. 25a zeigt eine für die Reservoir- oder Reglereinheit 43 gemäß Fig. 25 exemplarische Druck-Volumenkurve, die über einen bestimmten Volumenbereich IBV hinweg einen konstanten Druck DP herstellt. Somit kann aus dem expandierten Ballon 58 heraus Volumen zum Ballon 42 abfließen, ohne dass sich durch den Volumenabfluss im Reservoir 56 ein Druckverlust einstellt, das Druckplateau DP also verlassen wird. Für Anwendungen dieser Reglertechnologie bei Beatmungstuben soll der so einstellbare isobare Volumeneberich IBV in etwa dem frei ausgeformten Volumen des tracheal dichtenden Ballons 42 entsprechen.

Fig. 25b zeigt eine besondere doppelkammerige Reservoirballon-Anordnung, bei der zwei ballon-basierte Reservoirs 56 nach Fig. 25 an einem einzigen gemeinsamen Grundgehäuse 59 montiert sind. Die eine Kammer ermöglicht dabei einen trachealen Dichtungsdruck von 25 bis 30 mbar, während die andere die Einstellung eines Tamponadedrucks von 5 bis 15 mbar, beispielsweise für die sub-glottische Tamponade, wie in Fig. 23a beschrieben, ermöglicht.

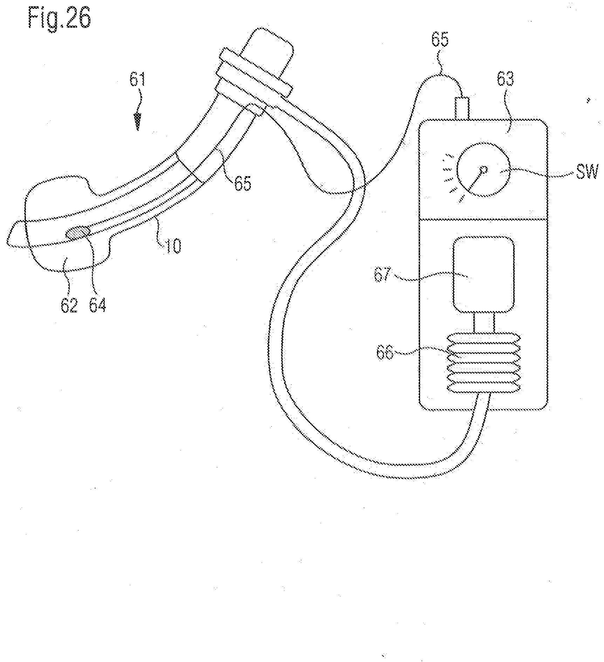

Fig. 26 zeigt einen Trachealtubus 61, der im Inneren des tracheal dichtenden Ballonsegmentes 62 mit einem druckaufnehmenden bzw. druckmessenden Fühlerelement 64 versehen ist. Der Druckfühler 64 ist in bevorzugter Ausführungsweise ein elektronisches Bauelement, welches sein Messsignal über eine Kabelleitung 65 an einen elektronisch gesteuerten Regler 63 weiterleitet. Das Fühlerelement 64 besteht vorzugsweise aus einem Absolutdrucksensor. Es können vorzugsweise Fühler auf der Basis von Dehnungsmessstreifen oder piezo-elektrische Sensoren verwendet werden. Der Regler 63 weist beispielsweise ein balgartiges oder kolbenartiges Reservoir 66 auf, welches durch einen Antrieb 67 betätigt, entweder Volumen zum Ballon 62 hin verschiebt oder aus dem Ballon 62 entnimmt. Der Antrieb 67 kann beispielsweise aus einem Schrittmotor bestehen oder als linear magnetischer Antrieb aufgebaut sein. Die Steuerung des Reglers 63 ist derart ausgelegt, dass Abweichungen des Fülldrucks im Bereich des dichtenden Ballonsegmentes 62 sofort durch eine entsprechende Volumenverschiebung kompensiert werden bzw. der Fülldruck auf einem am Regler 63 einstellbaren Sollwert SW konstant gehalten wird. Die Stabilisierung des dichtenden Ballondrucks erfolgt mit diesem Verfahren bereits zu einem Zeitpunkt, der optimal früh vor dem Einsetzen des maschinellen Beatmungshubes bzw. dem tatsächlichen Volumenfluss von Atemgas in die Lunge des Patienten liegt. Dies ist besonders bei Patienten relevant, die nach langer kontrollierter maschineller Beatmung erhöhte Atemarbeit aufwenden müssen, um eine nicht ausreichend volumendehnbare Lunge bis zu einem Punkt aufzuspannen, der einen effektiven Volumenstrom in die Lunge auslöst. In dieser Phase der isometrischen Anspannung der Lunge innerhalb des Thorax bzw. des mit der Anspannung der versteiften Lunge einhergehenden Druckabfalls innerhalb des Brustkorbs kann es zu aspirationsverursachenden Abfällen des Ballonfülldrucks kommen.

Im Gegensatz zu einem mechanischen Regler 43 einfachen Bautyps, welcher ein isobares Reservevolumen von vorzugsweise 20 bis 35 mbar zur Verfügung stellt, kann bei der beschriebenen elektronischen Regeleinheit 63 ein Druck aufgebaut werden, der den tracheal unkritischen Dichtungsdruck von 20 bis 35 mbar kurzzeitig überschreitet und so eventuellen vom Patienten ausgelösten Druckspitzen im trachealen Ballon dichtend entgegenwirken kann.

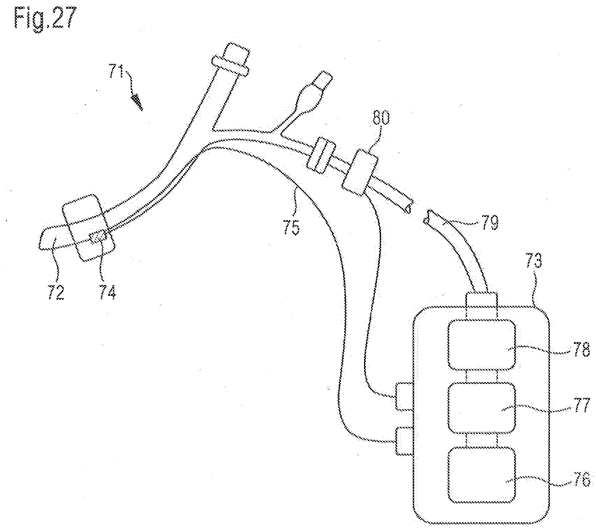

Fig. 27 zeigt ein rückgekoppeltes Reglersystem mit einem Regler 73, der an einen fluss-optimierten Trachealtubus oder -katheter 71 des erfindungsgemäßen Bautyps angeschlossen ist. Der Katheter 71 verfügt innerhalb des dichtenden Ballons 72 über einen dort fix integrierten elektronischen Drucksensor 74, der über eine Kabelverbindung 75 mit dem Regler 73 verbunden wird. Der Regler 73 selbst besteht aus einem Pumpen-Modul 76 mit optional integriertem Reservoir 77 und mindestens einem regelnden Ventil-Modul 78 mit integrierter Steuereinheit. Soll- und Alarmwerte können vom Anwender in die Steuerung eingegeben werden. Optional kann der Regler 73 auch über zwei Pumpensysteme, mit jeweils angeschlossenem Reservoir verfügen, wobei das eine Reservoir einen Überdruck und das andere einen Unterdruck vorrätig hält. Die Gradienten bzw. der im Regler 73 vorrätig gehaltene Differenzdruck zum Sollwert im Cuff 72 werden vom Regler 73 in einer optionalen Ausführung selbständig, durch einen lernenden Algorithmus derart eingestellt, dass die Latenz bis zum Erreichen des Sollwertes im Cuff 72 im Bereich von 10 bis 20 ms liegt. Sowohl die Pumpfunktionen als auch die Ventilfunktionen basieren bevorzugt auf piezoelektrischen Komponenten, die präzise und schnell, sowie leise und energiesparend betrieben werden können.

Die Zuleitung 79 zum extrakorporalen Anschluss des Katheters 71 weist bevorzugt einen inneren Durchmesser auf, der den Durchmesser des Schenkels überschreitet, und im idealen Fall um 30% überschreitet, um so widerstandsbedingte Strömungsverluste möglichst klein zu halten.

Alternativ zum cuff-integrierten Druck-Sensor 74 kann ein peripherer druckwandelnder Sensor 80 in die Zuleitung integriert sein, der in unmittelbarer Nähe zum Konnektor positioniert ist. Bei dieser Ausführung kann auf einen im Cuff 72 integrierten Sensor 74 verzichtet werden, wobei eine gewisse Verzögerung der Regelzeit in Kauf genommen wird.

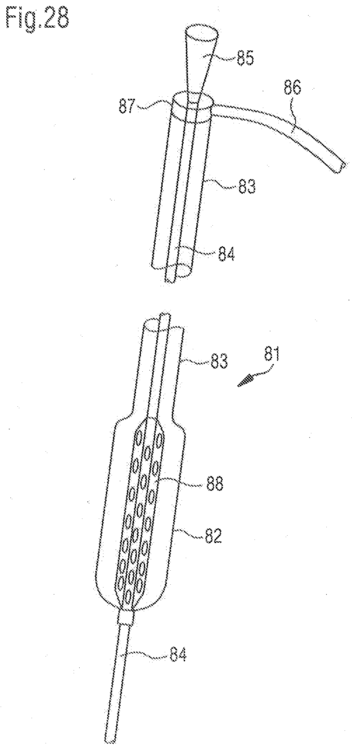

Fig. 28 zeigt eine trans-ösophageale Sonde 81 zur Zu- oder Ableitung von Substanzen oder Medien in den Magen oder duch den Magen hindurch, die mit einem ösophageal dichtenden Ballonelement 82 ausgestattet ist. Das proximale Ballonende 83 kann bis in den Bereich des extrakorporalen Konnektors 85 verlängert sein. Dort kann es in dicht schließender Weise mit einem auf dem Schaft 84 frei verschiebbaren dichtenden Abschlusselement 87 verbunden sein, welches wiederum in eine lumen-große Zuleitung 86 übergeht, welche an einen erfindungsgemäßen Regelmechanismus 43 angeschlossen ist. Die Reglerkomponente 43 stellt bei der ösophagealen Dichtung innerhalb des kommunizierenden Binnenraumes ein dichtendes Druckplateau DP von ca. 20 bis 30 mbar ein.

Zum verbesserten stationären Verbleiben des tamponierend dichtenden Ballonsegmentes im Ösophagus, kann dieses im osophagealen Bereich mit einem nicht kollabierbaren Profil 88 versehen werden, welches im Falle einer persiataltischen Kontraktion des Ösophagus Volumen aus den Ballonsegmenten vor der Kontraktionswelle, durch das oder unter dem Profil hindurch in Bereiche abgeleitet wird, die bereits von der Welle wieder frei gegeben sind. Somit kann ein Aufpilzen von Füllmedium vor der Kontraktionswelle verhindert werden, was den magenwärts gerichteten Transport der gesamten Vorrichtung zur Folge hätte. Entsprechende Profile sind in der bereits in der EP 0929339 B1 beschrieben und können im vollen dort offenbarten Umfang im Rahmen der Erfindung verwendet werden.

**Bezugszeichenliste**

| | | | |
|---|---|---|---|
| 1 | Ballonschlauchfolie | 21 | PVDC-Lage |
| 2 | Ballonanteil | 22 | PVC-Lage |
| 2a | distaler Ballonbereich | 23 | Invagination |
| 2b | mittiger Ballonbereich | 24 | stegartiges Element |
| 3 | Schaftschlauchanteil | 24a | Wandungslage |
| 3a | intra-rektaler Anteil | 24b | Wandungslage |
| 3b | trans-analer Anteil | 25 | ösenartige Formation |
| 3c | prä-analer Anteil | 26 | Eintrittsbereich |
| 4 | Kompartiment | 27 | kanalartige Formation |
| 5 | Kopfeinheit | 28 | Stirnseite |
| 6 | Schlauchanteil, Lumen | 29 | Graph |
| 7 | Wandungsprofil | 30 | Graph |
| 8 | U-förmige Erweiterung | 31 | Harnblasenkatheter |
| 9 | U-förmige Erweiterung | 32 | Schaft |
| 10 | Barriere-Lage | 33 | Ballon |
| 11 | Polyurethan-Lage | 34 | Öffnung |
| 12 | Polyamid-Lage | 35 | Band |
| 13 | Polyvinylchlorid-Lage | 36 | Band |
| 14 | äußere PUR-Lage | 37 | Öffnung |
| 15 | innere PUR-Lage | 38 | Trigonum vesicae |
| 16 | Barrierelage | 39 | innere Hamröhrenöffnung |
| 17 | Umkehrpunkt | 41 | Trachealtubus |
| 18 | Ballonschaftende | 42 | Dichtungsballon |
| 19 | TPE-Lage | 43 | Reservoir-, Reglereinheit |
| 20 | Vermittlerschicht | 44 | Schaftkorpus |
| 45 | Schaftende | 72 | Dichtungsballon |
| 46 | Zuleitung | 73 | Regler |
| 47 | Ventil-Drossel-Element | 74 | Drucksensor |
| 48 | Schaftprofil | 75 | Kabelverbindung |
| 49 | Zuleitungs-Lumen | 76 | Pumpen-Modul |
| 51 | Trachealtubus | 77 | Reservoir |
| 52 | Ballonelement | 78 | Ventil-Modul |
| 52a | Cuff | 79 | Zuleitung |
| 52b | Cuff | 80 | Druck-Sensor |
| 53 | zirkuläre Verjüngung | 81 | trans-ösophageale Sonde |
| 54 | Ventil | 82 | Ballonelement |
| 55 | Drossel | 83 | proximales Ballonende |
| 56 | Ballonblase | 84 | Schaft |
| 57 | Ruhefigur | 85 | Konnektor |
| 58 | Arbeitsfigur | 86 | Zuleitung |
| 59 | Sockelgehäuse | 87 | Anschlusselement |
| 60 | Einwegeventil | 88 | nicht kollabierbares Profil |
| 61 | Trachealtubus | D | distales Ballonende |
| 62 | Ballonsegment | GR | großer Radius |
| 63 | Regler | P | proximales Ballonende |
| 64 | Fühlerelement | R | Ebene |
| 65 | Kabelleitung | | |
| 66 | Reservoir | | |
| 67 | Antrieb | | |
| 71 | Trachealtubus | | |

## Patentansprüche

1. Vorrichtung zur minimal irritierenden, gewebeverträglichen, sich bevorzugt an die Bewegungen des Körpers und seiner Organe anpassenden Zu- und/oder Ableitung von Substanzen, umfassend einen in einem Binnenraum des Körpers platzierbaren, von außerhalb des Körpers befüllbaren Ballon (2), der - ggf. zusammen mit einem zu- und/oder ableitenden, den Ballon (2) tragenden Schlauch- oder Schaftsegment (3,3a) - ein befüllbares Kompartiment (4) umschließt, an welches sich ein schlauch- oder schaftförmiges Segment (3b) anschließt, das den Binnenraum mit der Körperoberfläche verbindet, wobei der Ballon (2) durch Blasformen aus einem Schlauchrohling aus einem mehrlagigen, folienartigen Material hergestellt ist, **dadurch gekennzeichnet, dass** der Schlauchrohling mehrlagig extrudiert ist, wobei in dem mehrlagigen Material zusätzlich zu wenigstens einer elastisch verformbaren Lage aus Polyurethan (PUR) wenigstens eine nicht elastisch verformbare, geruchs- und/oder mediendichte Barriere-Lage aus Ethylen-Vinylalkohol-Copolymer (EVOH) oder aus Polyvinylidenchlorid (PVCD), oder aus Polyamid (PA) oder aus einem thermoplastischen Polyamidelastomer (TPE-A) vorgesehen ist, und wobei der der Blasformung unterzogene Schlauchrohling in einem vorangehenden, getrennten Verfahrensschritt extrudiert worden ist, so dass sich in der Lage aus Polyurethan ein für das Blasformen erforderliches Verhältnis zwischen kristallinen und amorphen Materialanteilen einstellt.

2. Vorrichtung nach Anspruch 1, **dadurch gekennzeichnet, dass** die Gesamtstärke aller elastisch verformbaren Lagen wenigstens dem 1,5-fachen der Gesamtstärke aller nicht elastisch verformbaren, geruchs- und/oder mediendichte Barriere-Lagen entspricht.

3. Vorrichtung nach Anspruch 1 oder 2, **dadurch gekennzeichnet, dass** der Ballon (2) durch ein auf thermischer Umformung basierendes Blasformungsverfahren hergestellt ist, insbesondere indem ein zuvor, in einem separaten Herstellungsschritt (ko-) extrudierter, relativ kleinlumiger und dickwandiger Rohschlauch durch Beaufschlagung mit Blasdruck auf einen relativ größeren Arbeitsdurchmesser expandiert und dabei in einen relativ dünnwandigen Folienschlauch umgeformt ist, insbesondere indem der Rohschlauch in erhitztem Zustand in eine graduell oder schrittweise erwärmte, profilierte Formwandung hinein geschmiegt ist und schließlich bei hoher Temperatur, der temperierten Formwandung anliegend, ausgeformt wird, vorzugsweise wobei zur Sicherstellung der strukturellen Durchgängigkeit bzw. Unversehrtheit der Barrierelagen im durch thermische Umformung aus dem Schlauchrohling geblasenen Ballon beim Blasformen aus dem ggf. auf maximal das 1,5-fache Maß gestreckten Rohschlauch ein maximales radiales Streckungsverhältnis von 1 : 8 (in der Form fixierter, gestreckter Rohschlauch bei beginnender Ausformung des Ballons unter Blasdruck : größter ausgeformter Durchmesser) nicht überschritten wird, insbesondere auch ein maximales radiales Streckungsverhältnis von 1 : 5 nicht überschritten wird.

4. Vorrichtung nach einem der vorhergehenden Ansprüche, **dadurch gekennzeichnet, dass** die wenigstens eine PUR-Lage (2)
a) aus einem thermoplastischen PUR von einem Typ mit einer Wasseraufnahme nach DIN ISO 62 von 5% oder weniger besteht, bevorzugt mit einer Wasseraufnahme nach DIN ISO 62 von 2% oder weniger, und/oder
b) ergänzt wird durch andere, auch nichtelastische Material-Lagen, bspw. PVC- und PE-basierte Material-Lagen;
insbesondere wobei in dem mehrlagigen Material Lagen mit elastischen Verformungscharakteristika, wie sie bevorzugt thermoplastischen Polyurethane (TPU) bieten, anteilig überwiegen, bspw. thermoplastische, ester- und ätherbasierte Polyurethane, bevorzugt mit Shore-Härten in den Bereichen 80A bis 95A, sowie in dem Härtebereich von 55D bis 70D, oder in dem Härtebereich von 55D bis 65D;
und/oder wobei eine Lage (2) aus sich elastisch verformendem PUR-Material mit einer Lage (3) aus sich plastisch verformendem Material kombiniert ist, insbesondere aus plastisch verformbarem PVC.

5. Vorrichtung nach einem der vorhergehenden Ansprüche, **dadurch gekennzeichnet, dass**
a) die gesamte Wandstärke der Ballonhülle gleich oder kleiner ist als 50 µm, vorzugsweise gleich oder kleiner ist als 40 µm, insbesondere gleich oder kleiner ist als 30 µm; und/oder
b) dass das Verhältnis der anteiligen Wandstärke der wenigstens einen PUR-Lage (2) zu der anteiligen Wandstärke der wenigstens einen Lage (3) aus einem nicht elastischen Material, bspw. aus PVC, zwischen 1 : 1 und 1 : 5 liegt, vorzugsweise zwischen 1 : 2 und 1 : 4 liegt, insbesondere etwa bei 1 : 3.

6. Vorrichtung nach einem der vorhergehenden Ansprüche, **dadurch gekennzeichnet, dass** durch die Kombination wenigstens einer EVOH- oder PVDC-Lage (3) mit wenigstens einer PUR-Lage (2) die Migration von Fluiden, beispielsweise von Luft oder von polaren Flüssigkeiten, insbesondere von Wasser, durch die Wandung (1) der Ballonhülle (13) reduziert ist.

7. Vorrichtung nach einem der vorhergehenden Ansprüche, **dadurch gekennzeichnet, dass** der Schlauchrohling aus einem drei- oder mehrlagigen Material besteht, insbesondere wobei die Ballonhülle dreilagig ist.

8. Vorrichtung nach einem der vorhergehenden Ansprüche, **dadurch gekennzeichnet, dass** zwischen der elastisch verformbaren PUR-Lage (2) und der nicht elastisch verformbaren Lage (3), beispielsweise aus PVC, eine gas- und/oder wasserdampfdichte Barriere-Lage (10), vorzugsweise aus PVDC oder EVOH, angeordnet ist, vorzugsweise wobei die anteilige Wandstärke der nicht elastisch verformbaren Lage (3), beispielsweise aus PVC, größer ist als die anteiligen Wandstärken der elastisch verformbaren PUR-Lage (2) und/oder der gas- und/oder wasserdampfdichte Barriere-Lage (10), vorzugsweise aus PVDC oder EVOH, und/oder wobei vorzugsweise das Verhältnis zwischen der anteiligen Wandstärke der gas- und/oder wasserdampfdichten Barriere-Lage (10), vorzugsweise aus PVDC oder EVOH, und der anteiligen Wandstärke der nicht elastisch verformbaren Lage (3), bspw. PVC, zwischen 1 : 1 und 1 : 5 liegt, vorzugsweise zwischen 1 : 2 und 1 : 4, insbesondere etwa bei 1 : 3.

9. Vorrichtung nach einem der vorhergehenden Ansprüche, **dadurch gekennzeichnet, dass** die Wandung der sich an die Kopfeinheit anschließenden, ab- und/oder zuleitenden, extrakorporalen Schlauchelement
a) derart dünnwandig ist, dass es sich bei Krafteinwirkung von außen radial nach innen gerichtet einstülpt oder auch zu einer flachen, bandartigen Struktur kollabiert, sich bei nachlassender Krafteinwirkung von außen, spontan-elastisch aufrichtet bis zu einem zumindest teilweise offenen, partiell gerundeten Querschnitt; und/oder
b) es dem, den Zugangsweg zum Binnenraum bildenden, trans-luminalen Segment (3b) der Vorrichtung bei entsprechender Belastung durch die anliegenden Strukturen des Körpers ermöglicht, in den Zustand einer querschnittreduzierenden, elastisch wirkenden, radial gerichteten Faltung oder Invagination überzugehen, sowie sich aus dem entsprechend verformten, querschnittreduzierten Zustand, in elastischer Weise in seine Ausgangsform aufzurichten, wenn die dem trans-luminalen Segment (3b) von außen anliegenden Kräfte nachlassen bzw. sich das jeweilige, zum Binnenraum führende Lumen öffnet.

10. Vorrichtung nach einem der vorhergehenden Ansprüche, **dadurch gekennzeichnet, dass** sich bei der in situ-Platzierung eines residual, d.h. mit einem entlang des Ballonumfangs überschüssigen Ballonmaterial ausgeformten Ballonkorpus typische, in des Balloninnere invaginierte Einstülpungen (8) der überschüssigen, residualen Ballonhülle formieren, vorzugsweise wobei in das Balloninnere invaginierte Einstülpungen (8) querschnittlich ösenartigen Umschlagformationen (6) aufweisen, die sich vorzugsweise in Längsrichtung des Ballons (13), also zwischen dessen distaler und proximaler Stirnseite (9), als kanalartige Formationen (9) erstrecken bzw. fortsetzen, insbesondere wobei
a) die querschnittlich ösenartigen Umschlagformationen (6), die sich vorzugsweise in Längsrichtung des Ballons (13) als kanalartige Formationen (9) erstrecken bzw. fortsetzen, bei einem Fülldruck des Ballons (13) von 30 mbar einen Öffnungsdurchmesser zwischen 30 µm und 120 µm aufweisen, vorzugsweise einen Ösendurchmesser zwischen 40 µm und 80 µm, und/oder wobei
b) infolge der Kombination mit wenigstens einer Lage (3) aus einem nicht elastischen Material, bspw. aus PVC, der Öffnungsdurchmesser der ösenartigen oder kanalartigen Formation (6,9) reduziert ist gegenüber dem Öffnungsdurchmesser einer ösenartigen oder kanalartigen Formation (6,9) bei der reinen PUR-Lage (2) gleichen Materialtyps und gleicher Lagenstärke, und/oder wobei
c) wenigstens eine Lage (3) aus einem nicht elastischen Material, bspw. aus PVC, von einer plastischen, nicht-elastischen Beschaffenheit ist, derart, dass ihre flächige Verformung, Verbiegung oder Verwindung bei in situ wechselnden Fülldrucken des Ballons (13) einen dämpfenden Effekt auf die Öffnungskinetik von ösen- bzw. kanalartigen Formationen (8) ausübt, und/oder wobei
d) sich die elastisch bedingte Öffnung bzw. Aufweitung der Ösen bzw. Kanäle bei passagerem oder zyklisch schwankendem Druckabfall im Ballon infolge der Kombination wenigstens einer PUR-Lage (2) mit wenigstens einer Lage (3) aus einem nicht elastischen Material, bspw. aus PVC, verlangsamt, und/oder wobei
e) infolge der Kombination wenigstens einer PUR-Lage (2) mit wenigstens einer Lage (3) aus einem nicht elastischen Material, bspw. aus PVC, der elastische Aufrichtungseffekt im Bereich der ösen- oder kanalartigen Umschlagformationen (6,9) derart reduziert ist, dass die Querschnittsflächen der sekretleitenden, ösen- und kanalartigen Strukturen (6,9), im Gegensatz zu einer einfach-lagigen, elastischen Ballonfolie (2) nur aus PUR sowohl verkleinert sind als auch bei zyklischen Schwankungen des Ballonfülldrucks vermindert sind, und/oder wobei
f) die ösen- oder kanalartigen Umschlagformationen (6,9) ihre Dichtungseigenschaft gegenüber Fluiden, insbesondere gegenüber Flüssigkeiten, beibehalten, solange der Ballonfülldruck und/oder die unteren Grenzwerte der zulässigen Schwankungen des Ballonfülldrucks bei oder oberhalb von 5 mbar liegen, und/oder wobei
g) sich die Querschnittsflächen der ösen- oder kanalartigen Umschlagformationen (6,9) um nicht mehr als 25 % erhöhen, vorzugsweise nur um 20 % oder weniger, solange der Ballonfülldruck und/oder die unteren Grenzwerte der zulässigen Schwankungen des Ballonfülldrucks bei oder oberhalb von 5 mbar liegen, und/oder wobei
h) sich die Querschnittsflächen der ösen- oder kanalartigen Umschlagformationen (6,9) um nicht mehr als 25 % erhöhen, vorzugsweise nur um 20 % oder weniger, solange die Druckamplitude und/oder die Differenz zwischen den beiden Druckextrema des Ballonfülldrucks in einem Bereich zwischen 5 mbar und 30 mbar liegt.

11. Vorrichtung nach einem der vorhergehenden Ansprüche, **dadurch gekennzeichnet, dass** eine wellig ausgeformte, ring- oder wendelartige Profilgebung (7) der Wandung der zu- und/oder ableitenden Schlauchstruktur (3,3a), die den im jeweiligen Binnenraum oder Organ retinierenden Ballon (2) oder Ballonanteil (2a) trägt, es der zu- und oder ableitenden Schlauchstruktur (3,3a) ermöglicht, den im Binnenraum positionierten Anteil (3,3a) der Vorrichtung im Moment des Einführens querschnittreduziert radial einzufalten, sowie dem im retinierenden Ballon (2) herrschenden Fülldruck ohne Reduktion des zu- und/oder ableitenden Querschnitts durch seine spezifische Aufrichtungswirkung entgegenzuwirken, sowie sich aus dem radial verformten Zustand heraus, prompt in die bei der Herstellung vorgegebene Ausgangsform aufzurichten, und so die jeweilige Mündung des zu- und/oder ableitenden Lumens (6) zum Binnenraum offen zu halten, vorzugsweise wobei die wellig ausgeformte, ring- oder wendelartige Profilgebung (7) des Profils durch einen ein- oder mehrschichtigen Tauchprozess oder durch einen Spritzgussprozess hergestellt sind.

12. Vorrichtung nach einem der vorhergehenden Ansprüche, **dadurch gekennzeichnet, dass** sie zum Einführen in die Trachea, den Oesophagus, die Urethra oder das Intestinum geeignet ist.

13. Verfahren zur Herstellung einer Vorrichtung gemäß einem der vorhergehenden Ansprüche zur minimal irritierenden, gewebeverträglichen, sich bevorzugt an die Bewegungen des Körpers und seiner Organe anpassenden Zu- und/oder Ableitung von Substanzen, umfassend einen in einem Binnenraum des Körpers platzierbaren, von außerhalb des Körpers befüllbaren Ballon (2), der - ggf. zusammen mit einem zu- und/oder ableitenden, den Ballon (2) tragenden Schlauch- oder Schaftsegment (3,3a) - ein befüllbares Kompartiment (4) umschließt, an welches sich ein schlauch- oder schaftförmiges Segment (3b) anschließt, das den Binnenraum mit der Körperoberfläche verbindet, wobei der Ballon (2) durch Blasformen aus einem Schlauchrohling aus einem mehrlagigen, folienartigen Material hergestellt wird, **dadurch gekennzeichnet, dass** durch mehrlagige Extrusion ein Schlauchrohling aus mehren Lagen hergestellt wird, wobei in dem mehrlagigen Material zusätzlich zu einer elastisch wirkenden Lage aus Polyurethan (PUR) wenigstens eine geruchsdichte oder mediendichte Barriere-Lage aus Ethylen-Vinylalkohol-Copolymer (EVOH) oder aus Polyvinylidenchlorid (PVCD), oder aus Polyamid (PA) oder aus einem thermoplastischen Polyamidelastomer (TPE-A) vorgesehen ist, und dass dieser mehrlagige Schlauchrohling anschließend durch thermische Umformung in einem Blasformungsverfahren wenigstens partiell zu einem Ballon ausgeformt wird.

14. Verfahren nach Anspruch 13, **dadurch gekennzeichnet, dass** der koextrudierte, relativ kleinlumige und dickwandige Schlauchrohling im Verlauf der thermischen Umformung in seiner Längsrichtung gestreckt und vorzugsweise in gestrecktem Zutand fixiert wird, vorzugsweise wobei der koextrudierte, relativ kleinlumige und dickwandige Schlauchrohling, vorzugsweise in gestrecktem Zustand, durch Beaufschlagung mit Blasdruck auf einen relativ größeren Arbeitsdurchmesser expandiert und dabei in einen relativ dünnwandigen Folienschlauch umgeformt wird, wobei der Schlauch in eine graduell oder in Schritte temperierte profilierte Formwandung hinein geschmiegt wird und schließlich, der vollständig temperierten Formwandung anliegend, umgeformt wird.

15. Verfahren nach einem der Ansprüche 13 oder 14, **dadurch gekennzeichnet, dass**
a) der Schlauchrohling durch eine drei- oder mehrlagige Folienextrusion hergestellt wird, und/oder dass
b) wenigstens eine Barriere-Lage, bspw. aus EVOH oder PVDC oder PA oder TPE-A, mit wenigstens einer Träger-Lage, bspw. aus TPU, PUR oder PVC oder PE, koextrudiert wird, und/oder dass
c) die mehrlagige Extrusion durch gleichzeitige Zuführung mehrer unterschiedlicher Materialschmelzen zu einem einzigen, gemeinsamen Extrusionskopf erfolgt.

## Claims

1. A device for supplying and/or draining substances in a minimally irritating, tissue-compatible manner that is preferably adapted to the movements of the body and its organs, comprising a balloon (2), which may be placed in an interior space of the body and filled from outside the body, which balloon - optionally together with a supplying and/or draining tube or shaft segment (3,3a) carrying the balloon (2) - surrounds a fillable compartment (4), to which a tube or shaft-like segment (3b) is attached that connects the interior space to the body surface, wherein the balloon (2) is manufactured by blow molding from a tube blank made of a multi-layer film-like material, **characterized in that** the tube blank is extruded in multiple layers, wherein, in addition to at least one elastically deformable layer of polyurethane (PUR), at least one not elastically deformable, odor- and/or media-tight barrier layer of ethylene vinyl alcohol copolymer (EVOH) or of polyvinylidene chloride (PVCD) or of polyamide (PA) or of a thermoplastic polyamide elastomer (TPE-A) is provided in the multi-layer material, and wherein the tube blank that has undergone blow molding has been extruded in a previous separate method step, such that a ratio between crystalline to amorphous material proportions necessary for the blow molding process is established in the layer of polyurethane.

2. The device according to claim 1, **characterized in that** the total thickness of all elastically deformable layers corresponds to at least 1.5 times the total thickness of all not elastically deformable, odor- and/or media-tight barrier layers.

3. The device according to claim 1 or 2, **characterized in that** the balloon (2) is made by means of a blow molding process based on thermal forming, in particular, by applying blowing pressure onto a tube blank having a relatively small lumen and thick walls and previously (co-) extruded in a separate manufacturing step in order to expand said blank to a relatively larger operating diameter, thereby forming it into a relatively thin-walled film tube, in particular, by nestling the heated tube blank into a gradually or incrementally heated, profiled mold wall and shaping it at high temperature while resting against the tempered mold wall, preferably wherein a maximal radial stretch ratio of 1 : 8 is not exceeded during blow molding in the balloon blow molded from the tube blank maximally stretched, if necessary, 1.5 times by means of thermal forming (stretched tube blank fixed within the mold at the start of the molding of the balloon under blowing pressure : largest molded diameter), especially even a maximal radial stretch ratio of 1 : 5 is not exceeded.

4. The device according to any one of the preceding claims, **characterized in that** the at least one PUR layer (2)
a) is made of thermoplastic PUR of a type having a water absorption of 5% or less according to DIN ISO 62, preferably having a water absorption of 2% or less according to DIN ISO 62, and/or
b) is supplemented by other, also inelastic material layers, for example by material layers based on PVC and PE;
especially wherein layers having elastic deformation characteristics, such as those preferably provided by thermoplastic polyurethanes (TPU), are proportionally predominant in the multi-layered material, for example thermoplastic, ester- and ether-based polyurethanes, preferably having a Shore hardness in the range from 80A to 95A, as well as in the range from 55D to 70D or in the range from 55D to 65D;
and/or wherein a layer (2) made of elastically deforming PUR material is combined with a layer (3) made of plastically deforming material, preferably made of plastically deformable PVC.

5. The device according to any one of the preceding claims, **characterized in that**
a) the total wall thickness of the balloon envelope is equal to or smaller than 50 µm, preferably equal to or smaller than 40 µm, more preferably equal to or smaller than 30 µm; and/or
b) the ratio between the wall thickness proportion of the at least one PUR layer (2) and the wall thickness proportion of the not elastically deformable layer (3), for example PVC, is between 1 : 1 and 1 : 5, preferably between 1 : 2 and 1 : 4, more preferably around 1 : 3.

6. The device according to any of the preceding claims, **characterized in that** by combining at least one EVOH or PVDC layer (3) with at least one PUR layer (2) the migration of fluids, for example of air or polar liquids, in particular of water, through the wall (1) of the balloon envelope (13) is reduced.

7. The device according to any one of the preceding claims, **characterized in that** the tube blank is made of a three-layered or multi-layered material, especially wherein the balloon envelope is three-layered.

8. The device according to any one of the preceding claims, **characterized in that** a gas- and/or water vapor-tight barrier layer (10), preferably made of PVDC or EVOH, is arranged between the elastically deformable PUR layer (2) and the not elastically deformable layer (3), for example made of PVC, preferably wherein the proportional wall thickness of the not elastically deformable layer (3), for example made from PVC, is bigger than the proportional wall thickness of the elastically deformable PUR layer (2) and/or the gas- and/or water vapor-tight barrier layer (10), preferably made of PVDC or EVOH, and/or wherein preferably the ratio between the wall thickness proportion of the gas- and/or water vapor-tight barrier layer (10), preferably made of PVDC or EVOH, and the wall thickness proportion of the not elastically deformable layer (3), for example PVC, is between 1 : 1 and 1 : 5, preferably between 1 : 2 and 1 : 4, more preferably around 1 : 3.

9. The device according to any one of the preceding claims, **characterized in that** the wall of the supplying and/or draining extracorporeal tube segment adjacent to the head unit
a) has such a thin wall thickness that it folds in a radially inward direction or collapses into a flat, strip-like structure when an external force is applied, and when the applied external force diminishes, it spontaneously straightens in an elastic manner until it reaches an at least partially open, partially rounded cross section; and/or
b) allows the transluminal segment (3b) of the device that forms the access path to the interior space to transit into the state of an elastically operative, radially directed, cross-section reducing folding or invagination when a corresponding load is applied by the abutting structures of the body, and to straighten into its original shape from the deformed state with reduced cross section, when the external forces onto the transluminal segment (3b) diminish or the respective lumen leading to the interior space opens, respectively.

10. The device according to any one of the preceding claims, **characterized in that** upon the in situ placement of a residually formed balloon body, that is, a balloon body that is formed with excess balloon material along the balloon circumference, typical invaginations (8) of the excess residual balloon envelope invaginated into the balloon interior are formed, preferably wherein invaginations (8) that are invaginated into the balloon interior have turned-over formations (6) with an eyelet-like cross section, which preferably spread out or extend as channel-like formations (9) in the longitudinal direction of the balloon (13), that is, between the distal and proximal end-face sides (9) of the balloon, especially wherein
a) the turned-over formations (6) with an eyelet-like cross section, which preferably spread out or extend as channel-like formations (9) in the longitudinal direction of the balloon (13), have an opening diameter between 30 µm and 120 µm, preferably an eyelet diameter between 40 µm and 80 µm, at a filling pressure of the balloon (13) of 30 mbar, and/or wherein
b) as a result of the combination with at least one layer (3) made of a non-elastic material, for example of PVC, the opening diameter of the eyelet-like of channel-like formation (6, 9) is reduced compared to the opening diameter of an eyelet-like or channel-like formation (6, 9) with a pure PUR layer (2) of the same type of material and the same layer thickness, and/or wherein
c) at least one layer (3) made of a non-elastic material, for example made of PVC, is of a plastic, non-elastic nature, such that its flat deformation, bending or twisting has an attenuating effect on the opening kinetics of eyelet- or channel-like formations (8) when the filling pressure of the balloon (13) changes in situ, and/or wherein
d) the elastically caused opening and/or expansion of the eyelets and/or channels is slowed as a result of the combination of at least one PUR layer (2) with at least one layer (3) made of a non-elastic material, for example of PVC, when the pressure within the balloon is reduced transiently or in a cyclically fluctuating way, and/or wherein
e) as a result of the combination of at least one PUR layer (2) with at least one layer (3) made of an non-elastic material, for example of PVC, the elastic straightening effect in the region of the eyelet-like or channel-like turned-over formations (6, 9) is reduced such that the cross-sectional areas of the secretion conveying, eyelet- and channel-like structures (6, 9) are both reduced in size and minimized in case of cyclical variations of the balloon filling pressure, in contrast to a single-layered elastic balloon film (2) made of PUR only, and/or wherein
f) the eyelet- or channel-like turned-over formations (6, 9) retain their sealing properties against fluids, in particular against liquids, as long as at least one of the balloon filling pressure and the lower limits of the permissible variations of the balloon filling pressure remains at or above 5 mbar, and/or wherein
g) the cross-sectional areas of the eyelet- or channel-like turned-over formations (6, 9) do not increase by more than 25%, preferably only by 20% or less, as long as at least one of the balloon filling pressure and the lower limits of the permissible variations of the balloon filling pressure remains at or above 5 mbar, and/or
h) the cross-sectional areas of the eyelet- or channel-like turned-over formations (6, 9) do not increase by more than 25%, preferably only by 20% or less, as long as at least one of the pressure amplitude and/or the difference between the two pressure extremes of the balloon filling pressure remains in a range between 5 mbar and 30 mbar.

11. The device according to any one of the preceding claims, **characterized in that** a corrugated, annular or helical-like profile shape (7) of the wall of the supplying and/or draining tube structures (3, 3a) supporting the balloon (2) or balloon portion (2a) retained within the respective interior space or organ allows the supplying and/or draining tube structure (3, 3a) to radially fold, with reduced cross section, the portion (3, 3a) of the device positioned within the interior space during insertion and to counteract the filling pressure within the retaining balloon (2) without reducing the supplying and/or draining cross section by means of its specific straightening effect and to promptly straighten itself from the radially deformed state back into the initial shape specified during manufacture, and thereby to keep the respective mouth of the supplying and/or draining lumen (6) open towards the interior space, preferably wherein the corrugated, annular or helical profile shape (7) of the profile is manufactured by means of a single-layered of multi-layered immersion process or by means of an injection molding process.

12. The device according to any one of the preceding claims, **characterized in that** it is suitable for insertion into the trachea, the esophagus, the urethra or the intestine.

13. A method for producing a device according to one of the preceding claims for supplying and/or draining substances in a minimally irritating, tissue-compatible manner that is preferably adapted to the movements of the body and its organs, the device comprising a balloon (2), which may be placed in an interior space of the body and filled from outside the body, which balloon - optionally together with a supplying and/or draining tube or shaft segment (3, 3a) that carries the balloon (2) - surrounds a fillable compartment (4) adjacent to a tube or shaft-like segment (3b) that connects the interior space to the body surface, wherein the balloon (2) is produced by blow molding from a tube blank made of a multi-layer film-like material, **characterized in that** a tube blank made of a multi-layers is manufactured by multi-layered extrusion, wherein, in addition to at least one elastically deformable layer of polyurethane (PUR), at least one odor- and/or media-tight barrier layer of ethylene vinyl alcohol copolymer (EVOH) or of polyvinylidene chloride (PVCD) or of polyamide (PA) or of a thermoplastic polyamide elastomer (TPE-A) is provided in the multi-layer material, and **in that** this multi-layered tube blank is subsequently at least partially molded into a balloon by thermal forming in a blow molding process.

14. The method according to claim 13, **characterized in that** the coextruded tube blank with relatively small lumen and thick walls is stretched in its longitudinal direction during the thermal forming and is preferably fixed in the stretched state, preferably wherein the coextruded tube blank with relatively small lumen and thick walls is expanded to a relatively larger operating diameter by applying blowing pressure, preferably in the stretched state, and is thereby transformed into a relatively thin-walled film tube, wherein the tube is nestled into a gradually or incrementally heated, profiled mold wall and finally reshaped while resting against the completely tempered mold wall.

15. The method according to any one of claims 13 or 14, **characterized in that**
a) the tube blank is manufactured by means of three- or multi-layered film extrusion, and/or that
b) at least one barrier layer, for example made of EVOH or PVDC or PA or TPE-A, is coextruded with at least one support layer, for example made of TPU, PUR or PVC or PE, and/or that
c) the multi-layered extrusion occurs by simultaneously supplying a plurality of different material melts to a single, common extrusion head.

## Revendications

1. Dispositif d'administration et/ou d'évacuation de substances d'une manière peu irritante, compatible avec les tissus et de préférence adapté aux mouvements du corps et de ses organes, le dispositif comprenant un ballonnet (2), qui peut être placé dans un espace intérieur du corps et qui est rempli de l'extérieur du corps, lequel ballonnet, éventuellement conjointement avec un segment de tube d'administration et/ou d'évacuation ou un segment d'arbre (3, 3a) qui porte le ballonnet (2), entoure un compartiment (4) remplissable auquel est fixé un segment en forme de tube ou en forme d'arbre (3b) qui relie l'espace intérieur à la surface du corps, le ballonnet (2) étant produit par moulage par soufflage à partir d'une ébauche de tube constituée d'un matériau de type film multicouche, **caractérisé en ce que** l'ébauche de tube est extrudée en plusieurs couches, **en ce qu'**en plus d'au moins une couche élastiquement déformable de polyuréthane (PUR), au moins une couche barrière non élastiquement déformable, étanche aux odeurs et/ou aux fluides, d'un copolymère éthylène-alcool vinylique (EVOH) ou de chlorure de polyvinylidène (PVCD) ou de polyamide (PA) ou d'un élastomère polyamide thermoplastique (TPE-A) est dis-posée dans le matériau multicouche, et **en ce que** l'ébauche de tube qui a subi le moulage par soufflage a été extrudée dans une étape de procédé séparée précédente, de telle sorte qu'un rapport, requis pour le procédé de moulage par soufflage, entre des proportions de matériau cristallin à amorphe s'établit dans la couche de polyuréthane.

2. Dispositif selon la revendication 1, **caractérisé en ce que** l'épaisseur totale de toutes les couches élastiquement déformables correspond au moins à 1,5 fois l'épaisseur totale de toutes les couches barrières non élastiquement déformables, étanches aux odeurs et/ou aux fluides.

3. Dispositif selon la revendication 1 ou 2, **caractérisé en ce que** le ballonnet (2) est réalisé selon un procédé de moulage par soufflage sur la base de formage à chaud, en particulier selon lequel un tube brut d'une lumière d'un diamètre relativement petit et à paroi relativement épaisse, pré-(co)extrudé dans une étape de fabrication séparée, est dilaté par alimentation en pression de soufflage à un diamètre utile relativement plus grand et étant ainsi transformé en un tube en film à paroi relativement mince, en particulier **en ce que** le tube brut à l'état chaud est intégré dans une paroi de moule profilée, chauffée graduellement ou progressivement, puis formé à haute température, appliqué à la paroi de moule tempérée, de préférence **en ce que** pour assurer la cohérence structurelle et/ou l'intégrité des couches barrières dans le ballonnet soufflé à partir de l'ébauche de tube par formage à chaud lors du moulage par soufflage à partir du tube brut étiré, le cas échéant au maximum à 1,5 fois sa dimension, un rapport d'étirage radial maximal de 1 : 8 (tube brut étiré, fixé dans le moule au début du formage du ballonnet sous pression de soufflage: diamètre maximal formé) n'est pas dépassé, en particulier également **en ce qu'**un rapport d'étirage radial maximal de 1 : 5 n'est pas dépassé.

4. Dispositif selon l'une des revendications précédentes, **caractérisé en ce que** ladite couche PUR (2)
a) est constituée d'un PUR thermoplastique d'un type présentant une absorption de l'humidité selon la norme DIN ISO 62 inférieure ou égale à 5 %, de préférence une absorption de l'humidité selon la norme DIN ISO 62 inférieure ou égale à 2 %, et/ou
b) complétée par d'autres couches de matériaux non élastiques, par exemple des couches de matériaux à base de PVC et PE;
en particulier **en ce que** dans les couches de matériaux multicouche présentant des caractéristiques de déformation élastique, telles que celles offertes de préférence par des polyuréthanes thermoplastiques (TPU), prédominent proportionnellement, par exemple les polyuréthanes thermoplastiques à base d'esters et d'éthers, de préférence de duretés Shore dans des plages comprises entre 80A et 95A, ainsi que dans la plage de dureté de 55D à 70D, ou dans la plage de dureté de 55D à 65D;
et/ou **en ce qu'**une couche (2) constituée de matériau PUR se déformant élastiquement est combinée à une couche (3) en matériau se déformant plastiquement, en particulier en PVC plastiquement déformable.

5. Dispositif selon l'une des revendications précédentes, **caractérisé en ce que**
a) l'épaisseur totale de paroi de l'enveloppe de ballonnet est égale ou inférieure à 50 µm, de préférence égale ou inférieure à 40 µm, en particulier égale ou inférieure à 30 µm; et/ou
b) le rapport de l'épaisseur de paroi proportionnelle de ladite couche PUR (2) par rapport à l'épaisseur de paroi proportionnelle de ladite couche (3) constituée d'un matériau non élastique, par exemple constituée de PVC, est entre 1 : 1 et 1 : 5, de préférence entre 1 : 2 et 1 : 4, en particulier est approximativement de 1 : 3.

6. Dispositif selon l'une des revendications précédentes, **caractérisé en ce que** par la combinaison d'au moins une couche EVOH ou PVDC (3) avec au moins une couche PUR (2), la migration de fluides, par exemple d'air ou de liquides polaires, en particulier d'eau, est réduite par la paroi (1) de l'enveloppe du ballonnet (13).

7. Dispositif selon l'une des revendications précédentes, **caractérisé en ce que** l'ébauche de tube est constituée d'un matériau à trois couches ou multicouche, en particulier **en ce que** l'enveloppe du ballonnet est constituée de trois couches.

8. Dispositif selon l'une des revendications précédentes, **caractérisé en ce qu'**une couche barrière étanche au gaz et/ou à la vapeur d'eau (10), de préférence en PVDC ou EVOH, est disposée entre la couche PUR élastiquement déformable (2) et la couche non élastiquement déformable (3), par exemple en PVC, de préférence **en ce que** l'épaisseur de paroi proportionnelle de la couche non élastiquement déformable (3), par exemple en PVC, est supérieure aux épaisseurs de parois proportionnelles de la couche PUR élastiquement déformable (2) et/ou de la couche barrière étanche au gaz et/ou à la vapeur d'eau (10), de préférence en PVDC ou en EVOH, et/ou **en ce que** de préférence le rapport entre l'épaisseur de paroi proportionnelle de la couche barrière étanche au gaz et/ou à la vapeur d'eau (10), de préférence en PVDC ou en EVOH, et l'épaisseur de paroi proportionnelle de la couche non élastiquement déformable (3), par exemple en PVC, est comprise entre 1 : 1 et 1 : 5, de préférence entre 1 : 2 et 1 : 4, en particulier approximativement de 1 : 3.

9. Dispositif selon l'une des revendications précédentes, **caractérisé en ce que** la paroi de l'élément de tube extracorporel, d'évacuation et/ou d'administration, adjacent à l'unité de tête
a) est à paroi mince de telle sorte qu'il se retourne radialement de l'extérieur vers l'intérieur sous action d'une force ou qu'il s'affaisse en une structure plane, en forme de bande, se redresse spontanément de manière élastique jusqu'à une section transversale au moins partiellement ouverte et partiellement arrondie lorsque la force exercée de l'extérieur diminue; et/ou
b) il permet au segment transluminal (3b) du dispositif formant la voie d'accès à l'espace intérieur, sous l'effet d'une sollicitation correspondante exercée par les structures adjacentes du corps, de passer à l'état de pliage ou d'invagination réduisant la section transversale, à effet élastique, orienté radialement, ainsi que de se redresser de manière élastique à partir de l'état déformé correspondant réduisant la section transversale pour reprendre sa forme initiale, lorsque les forces exercées de l'extérieur sur le segment transluminal (3b) diminuent ou lorsque la lumière respective menant à l'espace intérieur s'ouvre.

10. Dispositif selon l'une des revendications précédentes, **caractérisé en ce que** lors du placement in situ d'un corps de ballonnet formé de manière résiduelle, c'est-à-dire avec un matériau de ballonnet excédentaire le long de la périphérie du ballonnet, des retournements (8) typiques, invaginés de l'enveloppe du ballonnet excédentaire résiduelle se forment à l'intérieur du ballonnet, de préférence **en ce que** des retournements (8) invaginés à l'intérieur du ballonnet présentent en section transversale des formations de rabat (6) de type œillet, qui s'étendent ou se prolongent de préférence dans le sens longitudinal du ballonnet (13), donc entre ses faces frontales (9) distale et proximale, sous forme de formations de type canal (9), en particulier **en ce que**
a) les formations de rabat de type œillet (6) en section transversale, qui s'étendent ou se prolongent de préférence dans le sens longitudinal du ballonnet (13) sous forme de formations de type canal (9), présentent un diamètre d'ouverture compris entre 30 µm et 120 µm à une pression de remplissage du ballonnet (13) de 30 mbar, de préférence un diamètre d'œillet compris entre 40 µm et 80 µm, et/ou
b) suite à la combinaison avec au moins une couche (3) en un matériau non élastique, par exemple en PVC, le diamètre d'ouverture de la formation de type œillet ou de type canal (6, 9) est réduit par rapport au diamètre d'ouverture d'une formation de type œillet ou de type canal (6,9) dans la couche PUR (2) pure du même type de matériau et de même épaisseur de couche, et/ou
c) **en ce qu'**au moins une couche (3) est constituée d'un matériau non élastique, par exemple de PVC, de nature plastique, non élastique, de telle sorte que sa déformation plane, sa flexion ou distorsion à des pressions de remplissage variables in situ du ballonnet (13) exerce un effet amortisseur sur la cinétique d'ouverture des formations de type œillet ou canal (8), et/ou
d) **en ce que** l'ouverture ou élargissement élastiquement conditionnel des œillets ou des canaux en cas de chute de pression passagère ou cycliquement fluctuante dans le ballonnet est ralentie suite à la combinaison d'au moins une couche PUR (2) avec au moins une couche (3) en un matériau non élastique, par exemple en PVC, et/ou
e) **en ce que**, suite à la combinaison d'au moins une couche PUR (2) avec au moins une couche (3) en un matériau non élastique, par exemple en PVC, l'effet de redressement élastique dans la zone des formations de rabat de type œillet ou canal (6, 9) est réduit de telle sorte que les surfaces de section transversale des structures de type œillet et canal (6, 9) acheminant des secrétions, contrairement à un film de ballonnet (2) élastique monocouche uniquement en PUR sont non seulement réduites mais aussi diminuées en cas de variations cycliques de la pression de remplissage du ballonnet, et/ou
f) **en ce que** les formations de rabat de type œillet ou canal (6, 9) conservent leur propriété d'étanchéité par rapport aux fluides, en particulier par rapport aux liquides, aussi longtemps que la pression de remplissage du ballonnet et/ou les valeurs limites inférieures des variations admissibles de la pression de remplissage du ballonnet sont de l'ordre de ou supérieures à 5 mbar, et/ou
g) **en ce que** les surfaces de section transversale des formations de rabat de type œillet ou canal (6, 9) n'augmentent pas plus de 25 %, de préférence seulement de 20 % ou moins, aussi longtemps que la pression de remplissage du ballonnet et/ou les valeurs limites inférieures des variations admissibles de la pression de remplissage du ballonnet sont de l'ordre de ou supérieures à 5 mbar, et/ou
h) **en ce que** les surfaces de section transversale des formations de rabat de type œillet ou canal (6, 9) n'augmentent pas plus de 25 %, de préférence seulement de 20 % ou moins, aussi longtemps que l'amplitude de pression et/ou la différence entre les deux extrêmes de pression de la pression de remplissage du ballonnet est/sont dans une plage comprise entre 5 mbar et 30 mbar.

11. Dispositif selon l'une des revendications précédentes, **caractérisé en ce qu'**un profilage (7) formé de manière ondulée, de type annulaire ou hélicoïdal de la paroi de la structure de tube d'administration et/ou d'évacuation (3, 3a), qui supporte le ballonnet (2) de rétention ou la partie de ballonnet (2a) dans l'espace intérieur respectif ou organe, permet à la structure de tube d'administration et/ou d'évacuation (3, 3a) de replier radialement, avec une section transversale réduite, la partie (3, 3a) position-née du dispositif dans l'espace intérieur au moment de l'introduction, ainsi que de contrer la pression de remplissage régnant dans le ballonnet (2) de rétention sans réduction de la section transversale d'administration et/ou d'évacuation par son effet de redressement spécifique, ainsi qu'à partir de l'état déformé radialement de se redresser rapidement dans la forme initiale prédéfinie lors de la fabrication, et ainsi de maintenir ouverte l'ouverture respective de la lumière d'administration et/ou d'évacuation (6) vers l'espace intérieur, de préférence **en ce que** le profilage formé de manière ondulée, de type annulaire ou hélicoïdal (7) du profil est réalisé par trempage monocouche ou multicouche ou par un procédé de moulage par injection.

12. Dispositif selon l'une des revendications précédentes, **caractérisé en ce qu'**il est adapté pour l'introduction dans la trachée, l'œsophage, l'urètre ou l'intestin.

13. Procédé de fabrication d'un dispositif selon l'une des revendications précédentes d'administration et/ou d'évacuation de substances d'une manière peu irritante, compatible avec les tissus et de préférence adapté aux mouvements du corps et de ses organes, le dispositif comprenant un ballonnet (2), qui peut être placé dans un espace intérieur du corps et qui est rempli de l'extérieur du corps, lequel ballonnet, éventuellement conjointement avec un segment de tube d'administration et/ou d'évacuation ou un segment d'arbre (3, 3a) qui porte le ballonnet (2), entoure un compartiment (4) remplissable auquel est fixé un segment en forme de tube ou d'arbre (3b) qui relie l'espace intérieur à la surface du corps, le ballonnet (2) étant produit par moulage par soufflage à partir d'une ébauche de tube constituée d'un matériau de type film multicouche, **caractérisé en ce qu'**une ébauche de tube est extrudée en plusieurs couches, **en ce qu'**en plus d'au moins une couche à effet élastique de polyuréthane (PUR), au moins une couche barrière étanche aux odeurset/ou aux fluides de copolymère éthylène-alcool vinylique (EVOH) ou de chlorure de polyvinylidène (PVCD) ou de polyamide (PA) ou d'un élastomère polyamide thermoplastique (TPE-A) est disposée dans le matériau multicouche, et que cette ébauche de tube multicouche est ensuite formée au moins partiellement en un ballonnet par formage à chaud selon un procédé de moulage par soufflage.

14. Procédé selon la revendication 13, **caractérisé en ce que** l'ébauche de tube coextrudée, de lumière d'un diamètre relativement petit et à paroi épaisse est étirée dans le sens de sa longueur au cours du formage à chaud et est de préférence fixée à l'état étiré, de préférence **en ce que** l'ébauche de tube coextrudée, de lumière d'un diamètre relativement petit et à paroi épaisse, de préférence à l'état étiré, est dilatée par alimentation en pression de soufflage à un diamètre utile relativement plus grand et étant ainsi transformée en un tube en film à paroi relativement mince, **en ce que** le tube est intégré dans une paroi de moule profilée, tempérée progressivement ou par étapes et finalement transformé en étant appliqué à la paroi de moule entièrement tempérée.

15. Procédé selon l'une des revendications 13 ou 14, **caractérisé en ce que**
a) l'ébauche de tube est réalisée par extrusion de film trois couches ou multi-couche, et/ou
b) **en ce qu'**au moins une couche barrière, par exemple en EVOH ou PVDC ou PA ou TPE-A, est coextrudée avec au moins une couche support, par exemple en TPU, PUR ou PVC ou PE, et/ou
c) **en ce que** l'extrusion multicouche s'effectue par l'amenée simultanée de plusieurs masses fondues différentes de matériaux dans une seule tête d'extrusion commune.
